# EUROPEAN PATENT APPLICATION

(11) **EP 4 813 408 A1**
(43) Date of publication of application: **30.09.2026**
(21) Application number: 25166540.2
(22) Date of filing: 27.03.2025
(51) Int. Cl.: A61K 40/11, A61K 40/31, A61K 40/42

(54) **CHIMERIC ANTIGEN RECEPTORS COMPRISING CYTOSOLIC CD4 COSTIMULATORY DOMAIN FOR IMMUNOTHERAPY**

(71) Applicant: Kemijski Institut, 1000 Ljubljana (SI)
(72) Inventor: JERALA, Roman, 1001 Ljubljana (SI); FINK, Tina, 1001 Ljubljana (SI); PLE KO, Ema, 1001 Ljubljana (SI)
(74) Representative: Weickmann & Weickmann PartmbB

(57) **Abstract**

This invention relates to a modified second-generation chimeric antigen receptor (CAR) that incorporates a cytosolic domain of the CD4 receptor as an additional or alternative costimulatory domain. In a second-generation CAR with the 41BB costimulatory domain the CD4 domain is inserted into the cytosolic tail adjacent to the transmembrane domain, either alongside or in place of the 41BB or CD28 domain in addition to the CD3zeta domain.

This modification described in this invention significantly enhances the performance of CAR constructs. In both in vivo and in vitro models targeting CD19, the CD4-modified CARs demonstrated superior T-cell activation and anti-tumor activity compared to clinically approved CARs that comprised 41BB as a costimulatory domain. Further testing with BCMA and Her2 (Erb2) targets confirmed the enhanced efficacy of the CD4-enhanced constructs demonstrating it as a platform versatile technology for targeting different targets and could be used also in combination with other targeting domains.

This invention offers a major advancement in CAR T cell therapy, providing a more potent and effective technology for cell immunotherapy by improving the costimulatory signaling.

## Description

### Field of the invention

This invention relates to the field of cell immunotherapy, particularly to chimeric antigen receptor (CAR) technology for cell-based therapies. The invention focuses on modifying the conventional structure of second-generation CAR by incorporating the cytosolic domain of the CD4 receptor as an additional or alternative costimulatory domain. The approach enhances the performance of therapeutic cells into which the CD4-containing CAR has been introduced through improved costimulatory signaling, resulting in more robust T-cell activation, persistence, and anti-tumor efficacy.

### Background

CAR T cell therapy has revolutionized treatment for hematologic malignancies by leveraging patient-derived T cells, which are genetically modified to express chimeric antigen receptors (CARs) that target specific tumor antigens and destroy cancer cells (Maher et al., 2002). The efficacy of CAR T cells heavily depends on the signaling provided by their costimulatory domains, which are essential for T-cell activation, persistence, and the effective elimination of cancer cells (Sadelain et al., 2009). Traditional second-generation CARs incorporate a primary costimulatory domain-typically CD28 or 41 BB-alongside the CD3ζ signaling domain. Each domain provides distinct advantages; CAR T cells with CD28 domains tend to initiate rapid activation and potent anti-tumor responses but are more susceptible to T-cell exhaustion (Van Der Stegen et al., 2015), while those with 41BB domains demonstrate slower activation but greater persistence and resilience against exhaustion (Cappell and Kochenderfer, 2021). In addition to cancer immunotherapy, CAR-based therapeutic cells can be used to treat various other diseases where eliminating a specific cell type plays a role in alleviating pathology. Examples include autoimmune diseases such as lupus and diabetes, targeting virus-infected cells, and addressing other conditions.

To further enhance CAR T cell function, research has explored alternative costimulatory domains, such as OX40 and ICOS, which have shown promise in improving cytotoxicity and promoting a phenotype conducive to T-cell persistence (Moreno-Cortes et al., 2023). However, limitations remain, as these modifications do not fully address issues related to suboptimal CAR T-cell activation and durability in certain cancers, especially in the context of solid tumors.

The CD4 receptor, a glycoprotein expressed on helper T cells, monocytes, macrophages, and dendritic cells, offers unique properties as a potential costimulatory domain. CD4 is a co-receptor that interacts with the T-cell receptor to recognize MHC Class II molecules and is essential for T-cell activation. The intracellular region of CD4 binds to the tyrosine kinase Lck, which initiates downstream signaling and supports T-cell activation by phosphorylation of Zap70 kinase that phosphorylates the ITAM domains of CD3zeta(Killeen and Littman, 1996; Kim et al., 2003). CD4 plays a major role primarily in CD4 helper T cells that do not kill target cells but stimulate production of cytokines upon engagement of the TCR. Cytotoxic CD8 T cells that are required to kill cancer cells, on the other hand, express CD8 and not CD4. Nevertheless, we reasoned that the ability of CD4 to recruit Lck may play a role in activation of CAR T cells and their ability to kill target cells that present at their membrane an antigen against which the CAR construct has been designed.

Therefore, it was very surprising when we observed that incorporation of the cytosolic domain of a CD4 receptor into the CAR construct, as the innovative step of this invention, strongly improved the therapeutic killing by CAR T cells, although killing is performed by cytotoxic CD8 T cells.

This invention builds on the CAR framework by incorporating the CD4 domain into second-generation CARs to improve the efficacy of therapeutic cells against both hematologic and solid tumors, addressing some of the limitations observed with existing costimulatory domains.

### SUMMARY OF THE INVENTION

This invention provides a new type of chimeric antigen receptor (CAR) that incorporates the cytoplasmic domain of the CD4 receptor as an additional or alternative costimulatory domain to enhance therapeutic CAR cell performance.

Using the CD4 intracellular domain provides improved therapeutic cells into which the genetic information for CD4-containing CAR has been introduced to improve activation, persistence, and anti-tumor efficacy.

The invention can be applicable across different CAR constructs with different targeting domains targeting various tumor antigens.

The invention covers all genetic constructs that comprise the intracellular CD4 domain but is implemented primarily in two primary configurations. In the first configuration, the cytosolic CD4 domain is positioned C-terminal to the transmembrane (TM) domain and followed by the CD3ζ signaling domain, where the CD4 serves as the sole costimulatory module instead of other often used domains, such as CD28 and 41BB. In the second configuration, CD4 is combined with the 41BB costimulatory domain, positioned N-terminal to the 41BB and CD3ζ, creating a dual-costimulatory effect to harness the benefits of both CD4 and 41BB signaling. Additional configurations with alternative CD4 placements further demonstrate the adaptability of CD4 positioning within the CAR structures. The intracellular CD4 domain could also be provided as a separate polypeptide chain to increase CAR activity due to proximity.

This invention can be applied for CARs targeting diverse tumor antigens, including CD19, Her2, and BCMA, demonstrating efficacy across diverse cancer types. The CD4-enhanced CAR T cells outperform conventional second-generation CARs with only 41BB and CD3ζ. The CD4-containing CARs exhibited enhanced activation, increased persistence, and improved cytotoxicity across all tested targets, confirming the versatility and effectiveness of CD4 as a universal costimulatory component.

This invention represents a significant advancement in CAR-based cell therapy by establishing the CD4 domain as a potent costimulatory domain that could be used in different implementations. It broadens the therapeutic application of CAR technology for cancer but also to other diseases, where the elimination of a select cell type is desired, making it a valuable tool for treating a range of cancers through improved therapeutic cell functionality and adaptability to different antigens.

Thus, improved CARs comprising the intracellular costimulatory CD4 domain, nucleic acids encoding such improved CARs and therapeutic cells comprising the CARs and/or nucleic acids encoding such improved CARs are provided herein alongside their therapeutic use.

### BRIEF DESCRIPTION OF THE FIGURES

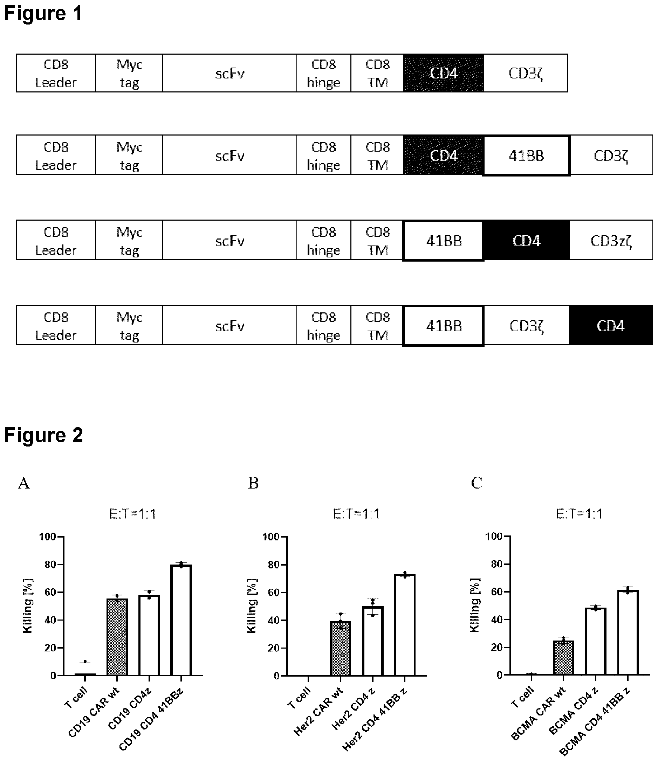

**Figure 1****: Scheme of CAR constructs with CD4 costimulatory domain**
   A scheme of CAR constructs comprising an antigen targeting scFv-domain, each incorporating the CD4 costimulatory domain at different positions relative to the 41BB and CD3ζ signaling domains. Antigen targeting is realized by selecting a suitable scFv sequence which is specific to the target antigen and different types of target cells, while the other elements of the CAR constructs remain unchanged.
**Figure 2****: Killing efficacy of CAR T cells against different targets**
   Comparison of tumor cell killing ability by CAR T cells comprising a CD4 costimulatory domain after 24 hours at an effector to target ratio of 1:1. (A) Targeting CD19 on RAJI cells. (B) Targeting Her2 on MCF7 ErbB2+ cells. (C) Targeting BCMA on RAJI cells.
**Figure 3****: Cytokine secretion of CAR T cells in response to different target antigens**
   Human IL-2 and IFN-γ levels in supernatants collected from co-cultures of CAR T cells with tumor cells at an effector to target ratio of 1:1 after 24 hours. (A) targeting CD19 on RAJI cells; (B) targeting Her2 on MCF7 ErbB2+ cells; (C) targeting BCMA on RAJI cells.
**Figure 4****: Killing efficacy of CAR constructs with alternative CD4 placements**
   Killing efficacy of CAR T cells expressing constructs with alternative CD4 placements after 24 hours at an effector to target ratio of 1:2 in co-cultivation with Raji cells.
**Figure 5****: In vivo survival and tumor bioluminescence analysis**
   (A) Experimental scheme outlining the in vivo study. Tumor models were established in NOD.CB17-Prkdc^{scid} IL2rg^{tm1}/BcgenHsd mice via intravenous injection of 1 × 10^6 RAJI-FLuc cells per mouse. After tumor establishment, CAR T cells (5 × 10^6 per mouse) were administered intravenously to evaluate the therapeutic efficacy. (B) The survival curve shows the percentage of animal survival over time after CAR T cell injection. (C) Total flux (p/s) over time, reflecting tumor burden through bioluminescent imaging of RAJI-FLuc cells.

### DEFINITIONS

The term **"cancer immunotherapy"** used herein relates to a treatment strategy that utilizes the body's immune system to combat cancer, often through immune cells, antibodies, or immune-modulating agents.

The term **"cell",** used herein, refers to a eukaryotic or prokaryotic cell, a cellular or multicellular organism (cell line) cultured as a single cell entity that has been used as a recipient of nucleic acids and includes the daughter cells of the original cell that has been genetically modified by the inclusion of nucleic acids. The term refers primarily to cells of higher developed eukaryotic organisms, preferably vertebrates, preferably mammals. This invention relies also on non-vertebrate cells, preferably plant cells.

The term **"cells"** also refers to human or animal primary cells or cell lines. Naturally, the descendants of one cell are not necessarily completely identical to the parents in morphological form and its DNA complement, due to the consequences of natural, random or planned mutations. A "genetically modified host cell" (also "recombinant host cell") is a host cell into which the nucleic acid has been introduced. The eukaryotic genetically modified host cell is formed in such a way that a suitable nucleic acid or recombinant nucleic acid is introduced into the appropriate eukaryotic host cell. The invention hereafter includes host cells and organisms that contain a nucleic acid according to the invention (transient or stable) bearing the operon record according to the invention. Suitable host cells are known in the field and include eukaryotic cells. It is known that proteins can be expressed in cells of the following organisms: human, rodent, cattle, pork, poultry, rabbits and the like. Host cells may include cultured cell lines of primary or immortalized cell lines.

The term **"T cell"** used herein relates to lymphocytes T, subset of white blood cells, a specific mononuclear immune cell population that interacts in adaptive immune system by recognizing antigen peptides bound to major histocompatibility complex molecules with T cell receptor. Recognition of antigen peptides via TCR activates signaling pathways which result in cytokine signaling and cytotoxic effect.

The term **"CAR"** used herein relates to "chimeric antigen receptor" that is transiently or stabilly expressed in T cells. CAR is a recombinant receptor, composed of the extracellular recognition domain, transmembrane domain and cytosolic activation domains that is localized at the T cell plasma membrane capable of recognizing tumor specific surface molecules independently of MHC and is used in cancer therapy. Upon specific recognition of the tumor surface molecules it triggers activation of a T cell. Recognition of specific antigen by CARs and T cell activation results in sequential killing of the tumor cells.

The term **"CAR wt"** used herein refers to a second generation CAR construct consisting of a single chain variable fragment (scFv), a 4-1BB costimulatory domain and a CD3ζ signaling domain. It serves as the standard unmodified CAR design used for comparison in experimental studies.

The term **"CAR T cells"** used herein relates to all T cells that bear coding sequence for CAR expression and have therapeutic effect of CAR T cells, in particular anticancer therapeutic effect.

The term **"CAR T cell therapy"** used herein relates to an immunotherapy approach wherein a patient's T cells are genetically modified to express a chimeric antigen receptor (CAR), enabling them to target and kill cancer cells.

The term **"recombinant"** used herein, means that a particular nucleic acid (DNA or RNA) is a product of various combinations of cloning, restriction and/or ligation or chemical synthesis leading to a construct having structurally coding or non-coding sequences different from endogenous nucleic acids in a natural host system.

The term **"transmembrane domain"** used herein relates to the hydrophobic region of a CAR construct that spans the lipid bilayer of the T cell membrane, allowing the extracellular domain to recognize antigens and the intracellular domain to transmit activating signals.

The term **"intracellular signaling domain"** used herein relates to the portion of the CAR construct located inside the T cell that transmits signals upon antigen binding, typically involving signaling pathways that lead to T cell activation, cytokine production, and cytotoxic responses.

The term **"costimulatory domain"** used herein relates to the intracellular signaling domain in CAR constructs that provides a secondary signal to enhance T cell activation, persistence, and functional responses, thereby promoting a more robust anti-tumor immune response in CAR T cell therapy.

The term **"CD4 receptor"** used herein relates to a glycoprotein expressed on the surface of T-helper cells and other immune cells, involved in the recognition of MHC Class II molecules and essential for T cell activation.

The term **"CD4 costimulatory domain"** used herein relates to the intracellular signaling portion of the CD4 receptor that binds to the tyrosine kinase Lck, facilitating downstream signaling pathways that enhance T cell activation and persistence, and is incorporated into CAR constructs to improve the efficacy of CAR T cells.

The term **"41BB costimulatory domain"** used herein relates to an intracellular signaling domain derived from the 41BB receptor, a member of the tumor necrosis factor receptor superfamily, that enhances T cell activation, survival, and memory formation, promoting sustained anti-tumor immunity in CAR T cell therapies.

The term **"identity"** refers to the sequence identity of nucleic acid or amino acid sequences. Identity determination can be done, for example, by a sequence alignment based on well-established and commonly used BLAST algorithms. Such an alignment is based on aligning similar nucleotide or amino acid sequences stretches with each other. Alignments, in particular multiple sequence comparisons, are typically done by using computer programs such as the Clustal series. Such comparisons allow determination of the similarity of the compared sequences. Said similarity is typically expressed in percent identity, i.e. the portion of identical nucleotides/amino acids at the same or corresponding (in an alignment) sequence positions relative to the total number of the aligned nucleotides/amino acids. For example, if in an alignment 90 amino acids of a 100 amino acid long query sequence are identical to the amino acids in corresponding positions of a template sequence, the sequence identity is 90%. The broader term "homology" additionally considers conserved amino acid substitutions, i.e. amino acids that are similar regarding their chemical properties, since those typically have similar chemical properties in a protein. Accordingly, such homology can be expressed in percent homology. If not indicated otherwise, sequence identity and sequence homology relate to the entire length of the aligned sequence.

The term **"scFv"** used herein relates to "single chain variable fragment" that is a recombinant antibody fragment that contains the variable regions of the heavy and light chains fused together into a single polypeptide chain, used in CAR constructs for antigen recognition.

The term **"single chain diabody"** as used herein refers to a recombinant antibody fragment composed of two single-chain variable fragments (scFvs) connected by short linker sequences that prevent intrachain pairing, enabling the formation of functional dimers. Single-chain diabodies may be bispecific, recognizing two different antigens, or monospecific, binding the same antigen with increased valency.

The term **"functional variant"** of a peptide or a peptide fragment, as used herein, refers to a peptide whose amino acid sequence has been modified compared to the original peptide, yet retains the essential biological functions of the original peptide or peptide fragment. Such modifications may include substitutions, deletions, or additions of amino acids that do not significantly impair its functionality. Thus, the functional variant retains at least 20%, at least 30%, at least 40%, at least 50%, at least 60%, at least 70%, at least 80%, at least 90%, or at least 95% of the functionality of the original peptide.

The term **"CD19"** used herein relates to a cell surface protein expressed on B cells and most B cell malignancies, which serves as a target for CAR T cells in the treatment of autoimmune diseases (e.g. systemic Lupus Erythematosus, Myasthenia gravis and Multiple sclerosis) and hematologic cancers like leukemia and lymphoma, as it is specifically expressed on tumor cells in these diseases.

The term **"Her2"** (Erb2) used herein relates to a receptor tyrosine kinase protein overexpressed in several cancer types, such as breast cancer, and is commonly targeted in CAR T cell therapies to eliminate tumors overexpressing this antigen, which plays a role in promoting tumor cell proliferation and survival.

The term **"BCMA"** used herein relates to B-cell maturation antigen, a cell surface protein expressed on plasma cells and some B cell-derived cancers like multiple myeloma, which serves as a target for CAR T cell therapies aiming to selectively kill malignant plasma cells.

The term **"GD2"** used herein relates to disialoganglioside GD2, a glycolipid antigen that is highly expressed on neuroectodermal-derived tumors, such as neuroblastoma, melanoma, and certain sarcomas, while being only weakly expressed on normal tissues, e.g. peripheral nerves. This expression pattern makes GD2 an attractive target for CAR T-cell therapies aiming to selectively eliminate malignant cells with minimal off-target effects.

The term **"target cell"** used herein relates to a cell, such as a tumor cell, that is specifically recognized and attacked by effector immune cells, such as CAR T cells, in therapeutic settings.

The term **"effector cell"** used herein relates to immune cells, such as CAR T cells, that are capable of directly attacking and killing target cells, such as cancer cells, by recognizing specific antigens on the tumor surface.

The term **"killing"** used herein relates to the process by which immune cells, including CAR T cells, induce apoptosis or direct cytotoxicity in tumor cells through mechanisms such as perforin/granzyme release.

The term **"persistence"** used herein relates to the ability of T cells to survive, proliferate, and maintain their functional activity over extended periods, which is critical for the long-term efficacy of CAR T cell therapy in eradicating tumors.

The term **"exhaustion"** used herein relates to the dysfunctional state of T cells after prolonged activation, characterized by a reduced ability to produce cytokines and kill target cells, commonly occurring in the context of chronic infections or cancer.

The term **"antigen recognition"** used herein relates to the binding of a specific antigen by a receptor, such as a CAR or TCR, on immune cells, which initiates signaling events that trigger immune responses such as T cell activation and tumor cell elimination.

The insertion of the vectors into the host cells is carried out by conventional methods known from the field of science, and the methods relate to transformation or transfection and include e.g.: chemically induced insertion, electroporation, microinjection, DNA lipofection, cellular sonication, gene bombardment, viral DNA input, as well as other methods. The entry of DNA may be of transient or stable. Transient refers to the insertion of a DNA with a vector that does not incorporate the DNA of the invention into the cell genome. A stable insertion is achieved by incorporating DNA of the invention into the host genome, where it can replicate with the cell and be transmitted to the progenitor cells. The insertion of the DNA of the invention, in particular for the preparation of a host organism having stably incorporated a nucleic acid, e.g. a DNA, of the invention, can be screened by the presence of markers. The DNA sequence for markers refers to resistance to antibiotics or chemicals and may be included on a DNA vector of the invention or on a separate vector.

The implementation examples that are described in more detail are designed to best describe the invention. These descriptions have no intention of limiting the scope of the invention and its applicability but are merely intended to provide a better understanding of the invention and its use.

### DETAILED DESCRIPTION OF THE INVENTION

The present invention introduces a novel type of a CAR construct that incorporates a cytosolic domain of the human CD4 receptor as an additional or alternative costimulatory domain to enhance therapeutic efficacy and cytotoxic response against tumor cells. CAR T cells are engineered to recognize and attack cancer cells through antigen specific engagement; however, current CAR T therapies can face challenges in maintaining sustained activity and efficient targeting across diverse tumor environments. By integrating a CD4 costimulatory domain within the CAR construct, this invention improves antitumor activity of CAR T cells targeting different antigens, including those found in haematological malignancies (e.g., CD19 and BCMA) and solid tumors (e.g., Her2, GD2). The primary application of this invention is in targeted cancer therapies, where enhanced CAR T cell functionality can provide more effective treatment options for patients with cancers such as leukemia, lymphoma, and certain solid tumors.

The present invention utilizes the intracellular domain of CD4, corresponding to sequences publicly available. One sequence is available in UniProt (Accession Number: P01730, Human CD4 of SEQ ID NO: 26). Another sequence derives from the work of Kim et al. (2003), where a single amino acid substitution was introduced: Methionine at position 407 in CD4 was replaced with Leucine to prevent internal digestion. This substitution was verified not to affect the formation of Lck/coreceptor complexes in heterologous expression systems.

Intracellular region of CD4 is characterized by its key functional elements that enhance intracellular signaling pathways. (Glatzová and Cebecauer, 2019):
1. Lck-Binding Site: The intracellular CD4 domain includes residues that bind lymphocyte-specific protein tyrosine kinase (Lck), enabling efficient recruitment of Lck to the CAR complex. Lck is a protein kinase enzyme essential for phosphorylating immunoreceptor tyrosine-based activation motifs (ITAMs) present in the CD3ζ chain of the CAR. This phosphorylation initiates downstream signaling cascades necessary for T cell activation and effector functions, such as cytokine production and cytotoxicity.
2. Palmitoylation Site: The CD4 intracellular domain contains palmitoylation motifs that facilitate its localization to lipid rafts in the plasma membrane. These microdomains are enriched in signaling molecules, further supporting optimal CAR signaling.
3. Basic-Rich Motif: This sequence aids in maintaining the structural integrity of the intracellular domain and possibly contributes to its localization and interaction with other signaling components.

By leveraging these intrinsic properties, the CD4 intracellular domain enhances CAR T cell signal transduction upon antigen engagement. This leads to amplified activation of pathways such as PI3K/AKT, NFAT, and NF-κB, resulting in robust T cell activation, proliferation, and sustained cytotoxicity against target cells.

Unlike traditional CAR constructs using costimulatory domains from CD28 or 41 BB, we found that the inclusion of the CD4 intracellular region surprisingly provides unique signaling kinetics. It allows more efficient recruitment of kinase activity directly to the CAR complex, thereby improving sensitivity to low antigen densities and enhancing cytotoxic efficiency (Van Der Stegen et al., 2015; Glatzová and Cebecauer, 2019).

Thus, in a first aspect a chimeric antigen receptor (CAR) is provided that comprises a CD4 intracellular domain, preferably a mammalian CD4 intracellular domain and even more preferably a human CD4 intracellular domain.

The intracellular CD4 domain has preferably, a length of at least 5 amino acids, more preferably at least 10 amino acids, more preferably at least 15 amino acids, more preferably at least 20 amino acids, more preferably at least 25 amino acids, more preferably at least 30 amino acids and even more preferably at least 35 amino acids. A length of 35 to 45 amino acids and in particular 38 amino acids is in particular preferred.

The inventive CAR preferably comprises the components:
(i) an extracellular antigen binding domain,
(ii) a hinge and transmembrane domain,
(iii) a CD4 intracellular co-stimulatory domain, preferably a mammalian CD4 intracellular co-stimulatory domain and even more preferably a human CD4 intracellular co-stimulatory domain and
(iv) an activating domain,
and wherein components (i) to (iv) are preferably comprised in N- to C-terminal order. A N- to C-terminal order of (i) to (iv) is in particular preferred.

The chimeric antigen receptor comprises preferably an antigen binding domain (i) comprising a functional binding fragment of a natural binding protein, in particular a receptor or antibody, preferably a single-chain variable fragment (scFv) of an antibody; or a short synthetic protein that binds to the selected antigen, such as machine learning-based designed binders (mini binder) or DARPINs. The antigen binding domain (i) is preferably of mammalian and in particular human origin.

The chimeric antigen receptor comprises preferably a hinge and transmembrane domain (ii) preferably comprising the hinge and transmembrane domain of CD8-alpha, and wherein the components (i) and (ii) are optionally joined by a short amino acid linker.

The chimeric antigen receptor comprises preferably an activating domain (iv) comprising a CD3ζ domain or CD3ε intracellular domain or another domain that comprises two or more ITAM motifs, which are phosphorylated upon recognition of cells with target antigens.

The chimeric antigen receptor comprises preferably a stimulatory domain (iii) comprising an additional N- or C-terminally bound co-stimulatory domain, preferably a 41BB domain.

The chimeric antigen receptor comprises preferably an activating domain (iv) which is interspersed between the two co-stimulatory domains of (iii), or, in configurations where the activating domain (iv) is N-terminally linked to component (ii), it is C-terminally linked to component (iii).

The chimeric antigen receptor comprises preferably an antigen binding domain (i) that specifically binds an antigen on the surface of a pathologic cell, preferably a tumor-associated antigen selected from CD19, BCMA, GD2 or ErbB2 or other target cell-specific antigens.

The chimeric antigen receptor comprises preferably the intracellular CD4 co-stimulatory domain (iii) comprising the amino acid sequence of SEQ **ID** NO: 21 or SEQ **ID** NO: 27 or a functional variant thereof having at least 75% sequence identity.

The chimeric antigen receptor comprises preferably a CAR comprising the amino acid sequences of any one of SEQ **ID** NOs 28-39, and 42-45 or functional variants thereof having at least 75% sequence identity, and optionally variants thereof lacking the leader peptide and/or the Myc tag.

A nucleic acid is provided encoding the inventive chimeric antigen receptor (CAR) fusion proteins claimed, wherein the nucleic acid encodes any one of SEQ **ID** NOs: 28-39, and 42-45 or functional variants thereof, in particular nucleic acids of SEQ **ID** NOs: 1-4, or nucleic acids having at least 75% sequence identity thereto.

A recombinant expression vector comprising the inventive encoding nucleic acids is provided, wherein the expression vector is optionally a viral vector.

An isolated mammalian cell comprising the inventive expression vector or being engineered to express the inventive encoding nucleic acid is provided. The corresponding host cell is preferably selected from the group consisting of an αβ T cell, a γδ T cell, a natural killer (NK) cell, a natural killer T (NKT) cell, a cytotoxic T lymphocyte (CTL), a regulatory T cell, macrophage or combinations thereof.

A pharmaceutical composition comprising the CAR, the nucleic acid or recombinant vector, or the isolated mammalian cell or population thereof, all as described above, and a pharmaceutically acceptable carrier and/or excipient is provided.

A method of treating, preventing, or ameliorating a condition or disorder associated with the expression of an antigen, preferably a tumor antigen, in a subject is provided. The method comprises administering to the subject an effective amount of the CAR, and/or the nucleic acid or recombinant vector, and/or the isolated mammalian cell or population thereof, and/or the pharmaceutical composition, all provided as described above.

The CAR, and/or the nucleic acid or recombinant vector, and/or the isolated mammalian cell or population thereof, and/or the pharmaceutical composition, all as described above are provided for use in treating, preventing and/or ameliorating a condition or disorder associated with the expression of an antigen, preferably a tumor antigen.

In a preferred embodiment the intracellular co-stimulatory CD4 domain is located C-terminal to the transmembrane domain of CD4 and spans from amino acid C419 to I458 of SEQ ID NO: 26. For the purpose of the invention, a variant shortened by two amino acids at the N-terminal end (ARG421 - ILE458 according to SEQ ID NOs: 21 or 27) is sufficient to provide the functionality required for co-stimulation, in particular the Lck-binding site, the palmitoylation site, and a basic-rich motif.

For the purpose of the invention, co-stimulatory CD4 domains encompass all variants that differ from the intracellular CD4 domain by amino acid substitutions, insertions, or deletions of one or more amino acids, provided they retain at least a functional Lck-binding site and, optionally, a functional palmitoylation site and/or a functional basic-rich motif.

Preferably, functional co-stimulatory CD4 variants have an amino acid sequence identity or homology, preferably identity, of at least 75%, at least 80%, 81 %, 82%, 83%, 84%, 85%, 86%, 87%, 88%, 89%, 90%, 91%, 92%, 93%, 94%, 95%, 96%, 97%, 98%, or 99% with SEQ ID NOs: 21 or 27.

In preferred embodiments the herein disclosed CAR comprises the intracellular co-stimulatory domain according to SEQ ID NO: 21.

In further preferred embodiments the herein disclosed CAR comprises the intracellular co-stimulatory domain according to SEQ ID NO: 27.

In the context of the herein disclosed CAR of the invention, the extracellular antigen-binding domain (i) refers to a functional "antigen-binding fragment" of an antibody, which is defined as a portion of an intact antibody that retains the ability to bind the same antigen as the intact antibody. Examples of antibody fragments include, but are not limited to, Fv, Fab, Fab', Fab'-SH, F(ab')₂; diabodies; linear antibodies; nanobodies (e.g., VHH); single-chain antibody molecules (e.g., scFv or single-chain diabodies); and multi-specific antibodies formed from antibody fragments.

In the context of the herein disclosed CAR of the invention, the extracellular antigen-binding domain (i) may also be a natural binding protein or functional fragment thereof, such as a receptor or a functional variant of the binding domain of a receptor. Alternatively, it may be a short protein with specific binding activity, such as a Designed Ankyrin Repeat Protein (DARPin), which consists of engineered ankyrin repeat motifs that form a stable, high-affinity binding scaffold, or a designed minibinder.

As used herein, a 'designed minibinder' refers to a computationally designed polypeptide that specifically binds to a target molecule, such as an antigen, through a structurally optimized binding interface. Unlike naturally occurring binding proteins, designed minibinders are generated *de novo* using structure-based computational design methods to achieve high binding affinity, specificity, and stability. These polypeptides typically adopt a well-defined three-dimensional fold, such as a helical bundle or β-sheet architecture, and may be engineered to resist denaturation and proteolytic degradation. Methods for providing minibinders are known in the art, e.g. WO2016005969A1.

In the context of the CAR disclosed herein, antibody-binding fragments are designed such that all sequences required for antigen binding, for example, the variable light chain (VL) and the variable heavy chain (VH) of an antibody-binding site, are arranged on a single polypeptide chain (single-chain). Accordingly, the antigen-binding domain (i) is preferably configured as an scFv, single-chain diabody, or nanobody.

In preferred embodiments, the antigen-binding domain (i) is an scFv fragment.

In the context of the herein disclosed CAR the hinge and transmembrane domain (ii) may be selected from the group comprising the transmembrane region(s) of) the alpha, beta or zeta chain of the T cell receptor, CD28, CD3 epsilon, CD45, CD4, CD5, CD8, CD9, CD 16, CD22, CD33, CD37, CD64, CD80, CD86, CD 134, CD137, CD154.

Alternatively, the transmembrane domain can be synthetic, in which case it will comprise predominantly hydrophobic residues such as leucine and valine. In several embodiments, a triplet of phenylalanine, tryptophan and valine will be found at each end of a synthetic transmembrane domain.

The inventive CAR molecules preferably comprise the hinge and transmembrane domain of CD8α.

The hinge and transmembrane domain (ii) link the extracellular antigen binding domain (i) to the intracellular co-stimulatory and/or signaling moiety of the CAR. On its N-terminal end the hinge moiety may be either joined directly to the C-terminal end of the antigen binding fragment (i) or joined via a short amino acid sequence having a length of e.g.1-50 amino acids.

To facilitate optimal binding of the antigen-binding domain (i) to sterically complex targets, for example, those on the surface of cancer cells, the hinge moiety in component (ii) is preferably configured as a flexible spacer. This spacer is preferably a naturally occurring or synthetic peptide with a length of up to 300 amino acids, for example, 10 to 200 amino acids, preferably 15 to 100 amino acids, and more preferably 25 to 50 amino acids.

In other embodiments, component (ii) is configured without a spacer.

Without being bound by theory, it is believed that suitable naturally occurring spacers are flexible protein domains that allow optimal orientation of the antigen binding domain, for example fragments of the Ch2-CH3 domains of IgG antibodies.

Other suitable naturally occurring spacers include hinge fragments of membrane-anchored T-cell proteins, such as CD4, CD8, CD28, or CD3-epsilon, or functional variants thereof.

In the context of the present invention, the hinge region of component (ii) is preferably the hinge region of the human CD8 receptor. In preferred embodiments, the hinge moiety of component (ii) is the human CD8α hinge region according to SEQ ID NO: 17 or a functional variant thereof.

The transmembrane domain of component (ii) serves as the structural anchor, integrating the receptor into the T-cell membrane. It ensures stability and proper orientation of the extracellular and intracellular domains and contributes to signal transduction by facilitating the spatial assembly of the CAR complex and associated signaling molecules.

In embodiments with a hinge moiety, the transmembrane domain is bound N-terminally to the hinge moiety. In embodiments without a hinge moiety, the transmembrane domain may also be directly bound to the antigen-binding component (i). At its C-terminal end, the transmembrane domain is bound to the intracellular co-stimulatory domain or the T-cell activating domain located on the cytoplasmic side of the plasma membrane.

The transmembrane domain can be derived either from a natural or from a synthetic source. Where the source is natural, the domain can be from any membrane-bound or transmembrane protein. Exemplary transmembrane domains for use in the disclosed CARs can include at least the transmembrane region(s) of) the alpha, beta or zeta chain of the T cell receptor, CD28, CD3 epsilon, CD45, CD4, CD5, CD8, CD9, CD 16, CD22, CD33, CD37, CD64, CD80, CD86, CD 134, CD137, CD154.

Alternatively, the transmembrane domain can be synthetic, in which case it will comprise predominantly hydrophobic residues such as leucine, isoleucine, and valine.

Selecting a suitable transmembrane domain might consider its compatibility with the intracellular signaling domain. The CD3ζ transmembrane domain (CD3ζ-TM) is particularly suitable when paired with its natural partner, the CD3ζ signaling domain, ensuring highly efficient signal transduction. However, the CD8 transmembrane domain and the CD28 transmembrane domain are more preferably selected, as they promote more stable surface expression of the CAR and work efficiently in combination with co-stimulatory domains such as CD28 or 4-1BB. These combinations support robust activation and extend the lifespan of CAR-T cells, making them particularly suitable for clinical applications.

In the context of the invention, it is disclosed that the CD8α transmembrane domain can also be used in combination with the co-stimulatory CD4 domain, in addition to the aforementioned domains. This applies regardless of whether the co-stimulatory CD4 domain is directly fused to the C-terminal end of the transmembrane domain or connected to the transmembrane domain via an additional polypeptide domain (e.g., a co-stimulatory 4-1BB domain).

In preferred embodiments, the transmembrane domain of component (ii) is the transmembrane domain of CD8α according to SEQ ID NO: 19 or a functional variant thereof.

In the context of the present invention, component (ii) comprises the hinge region according to SEQ ID NO: 17 as well as the transmembrane domain according to SEQ ID NO: 19, including their functional variants.

In the context of the herein disclosed CAR, component (iv) is an activating domain.

The disclosed CARs often include primary cytoplasmic signaling sequences that act in a stimulatory manner, which may contain signaling motifs that are known as immunoreceptor tyrosine-based activation motifs or ITAMs. Examples of ITAM containing primary cytoplasmic signaling sequences that can be included in a disclosed CAR include those from CD3ζ, FcRγ, CD3 γ, CD3δ, CD3ε, CD79(a or b), and CD66d proteins.

In preferred embodiments, the cytoplasmic activating domain of the CAR may comprise an intracellular T cell signaling domain from CD3ζ. It is preferred that the activating CD3ζ domain comprises three ITAM motifs, corresponding to its naturally occurring form. Depending on the application, the CD3ζ domain may alternatively comprise only one or two ITAM motifs to elicit a less intense T-cell activation, for example.

It is preferred that the activating domain (iv) of the CAR comprises the amino acid sequence of SEQ ID NO:25 or a functional variant thereof.

In specific embodiments, it is further preferred that the cytoplasmic activating domain of the CAR comprises the signaling domain from CD3ε or a functional variant thereof.

The disclosed CAR is a single-chain polypeptide comprising, in an N-terminal to C-terminal order, the following components: (i) an extracellular antigen-binding domain, (ii) a hinge and transmembrane domain, (iii) a CD4 intracellular co-stimulatory domain, and (iv) an activating domain. To ensure proper processing and translocation to the cell membrane, preferred embodiments provide for a leader peptide (signal peptide) at the N-terminal end of this polypeptide. This leader peptide may optionally comprise a short sequence, preferably 1-50 amino acids in length, which might be realized as a tag (e.g. Myc tag). The leader sequence is not specifically limited, as a variety of known leader sequences from transmembrane proteins are suitable.

In preferred embodiments, the CD8 leader sequence according to SEQ ID NO: 6, or a functional variant thereof, is selected.

In preferred embodiments, a CARs disclosed herein is designed such that component (iii) comprises an additional N- or C-terminally linked co-stimulatory domain.

As mentioned above, second-generation CARs combine the CD3ζ signaling domain with a co-stimulatory domain, which together govern key aspects of CAR T-cell functionality, including activation, proliferation, and persistence. The therapeutic efficacy of these CAR T cells is therefore largely determined by the selected co-stimulatory domain, as it plays a critical role in modulating the immune response thereby ensuring the effective elimination of target cells, such as tumor cells.

In addition to the improved second-generation CAR with a CD4 co-stimulatory domain presented in this document, co-stimulatory domains CD28 and 4-1BB (CD137) typically form the foundation of therapeutic 2^{nd} generation CAR designs. The CD28 domain induces rapid and robust T-cell activation, while the 4-1BB domain ensures prolonged T-cell persistence and resistance to exhaustion.

Furthermore, expanding the repertoire of co-stimulatory domains has shown to offer additional therapeutic opportunities. OX40 (CD134) enhances the long-term survival and function of T cells, making it relevant for applications focused on sustained effects. ICOS promotes a balanced T-cell response and plays a critical role in the differentiation of helper T cells. HVEM amplifies cytotoxic activity and T-cell persistence, potentially increasing effectiveness in tumor models. CD27 supports T-cell activation and proliferation, particularly in therapeutic approaches aimed at eliciting rapid and robust immune responses. GITR fosters both proliferation and functionality of T cells, especially in combination with other signaling pathways, offering a versatile option for CAR T-cell optimization.

The targeted selection and integration of these co-stimulatory domains promises precise adaptation of CAR T cells to specific clinical requirements, thus contributing to the enhancement of the therapeutic efficacy and safety of related treatments.

In the context of the present invention, the additional co-stimulatory domain optionally included in component (iii) is not limited to the corresponding domains of the aforementioned CD28, 4-1BB, OX40, ICOS, HVEM, CD27, and GITR, or their functional variants. Rather, a skilled person may identify additional advantageous co-stimulatory domains, for example, based on their effects on T-cell proliferation and/or persistence.

In preferred embodiments, the additional co-stimulatory domain of component (iii) is selected from the co-stimulatory domains of the proteins CD28 or 4-1BB, preferably 4-1BB.

In specific embodiments the activating domain (iv) is interspersed between two co-stimulatory domains (iii). Alternatively, in configurations where the C-terminus of the activating domain (iv) is N-terminally linked to component (ii), its C-terminus is further linked to component (iii).

Co-stimulatory domains influence key factors such as proliferation, persistence, and cytokine release in CAR T cells in distinct ways, thereby contributing in a differentiated manner to the therapeutic success of CAR T-cell therapies. Surprisingly, CAR T cells with a co-stimulatory domain derived from CD4 benefit from the incorporation of an additional co-stimulatory domain, specifically through its integration into the protein sequence of the CAR.

Preferably, this additional co-stimulatory domain is integrated into component (iii) of the CAR, either at the N-terminus or at the C-terminus of the CD4 domain. The additional domain is either attached directly to the CD4 domain or linked via a short linker that is preferably 1-50 amino acids in length. Depending on the integration site, the additional domain is either positioned N-terminally to the transmembrane domain of component (ii) or linked to the CD4 domain. In the latter case, the additional domain is located between the CD4 domain and the activation domain (iv) within the amino acid sequence of the CAR.

In preferred embodiments the additional co-stimulatory domain is integrated between the CD4 domain and the activation domain.

In embodiments where the activation domain is directly linked to the transmembrane domain, the additional co-stimulatory domain is either positioned at the C-terminal end of the CAR or integrated between the activation domain (iv) and the CD4 domain.

Combining the CD4 domain with an additional co-stimulatory domain is not limited to a specific domain; rather, the skilled person can select an appropriate domain based on specific requirements. However, preferred embodiments include those containing a CD28 domain or a 4-1BB domain in addition to the CD4 domain.

More preferably, embodiments include a 4-1BB domain alongside the CD4 domain.

Four basic domain configurations of CAR constructs are disclosed in Figure 1, each designed to optimize signaling and therapeutic efficacy.

In the first configuration, the CD4 intracellular domain replaces the 41BB costimulatory domain, situated directly between the CD8 transmembrane domain and the CD3ζ signaling domain. In the second configuration, the CD4 intracellular domain is introduced downstream of the CD8 transmembrane domain and upstream of the 41BB costimulatory domain. In the third configuration, the CD4 intracellular domain is positioned downstream of the 41BB costimulatory domain and upstream of the CD3ζ signaling domain. In the fourth configuration, the CD4 intracellular domain is located terminally, downstream of the CD3ζ signaling domain.

The data from Experiments 3-5 demonstrate that CAR T cells benefit synergistically, and regardless of the chosen configuration, from the combination of the CD4 domain with an additional co-stimulatory domain. This effect is particularly pronounced when 4-1BB is selected as the co-stimulatory partner.

The effectiveness of CAR T-cells lies in their ability to specifically recognize target cells and eliminate them by employing one or more mechanisms inherent to or triggered by the CAR T-cell. This includes direct cytotoxicity (e.g., granzyme-induced apoptosis), cytokine release, and the recruitment of additional immune cells, making CAR-T therapies a highly effective treatment for proliferative diseases, particularly hematological malignancies.

A critical aspect of CAR T-cell-mediated elimination of target cells lies in their highly specific antigen recognition, which in turn relies on the incorporated antibody-derived antigen-binding fragments (typically single-chain polypeptides comprising the variable domains, preferably scFvs) within the CAR structure. These scFvs are designed to bind with high specificity to predefined target antigens expressed on the surface of a malignant cell or a specific cell type. This antigen-specific recognition not only triggers T-cell activation but also minimizes off-target effects, a crucial factor for the safety and efficacy of CAR-T therapies.

The modular structure of CARs enables the simple integration of antigen-binding fragments for a broad repertoire of already validated target antigens. This adaptability leverages extensive research in the field of antibodies, enabling a skilled person to tailor CAR-T cells for diverse therapeutic applications.

In the context of the present invention, the specificity of the antigen-binding domain (i) is not restricted. Rather, a skilled person can select from a variety of antigen-binding domains when designing an appropriate CAR, preferably focusing on antibody fragments that have been therapeutically validated. Attractive antigens within the scope of this invention include tumor-associated antigens (TAAs) as well as antigens expressed on autoreactive cells, such as autoreactive B cells. A non-limiting list of TAA that are predominantly associated with solid tumors comprises Erb2 (HER2/neu), EGFR, EGFRvIII, MUC1, mesothelin, CEA, PSMA, GD2, GPC3, NY-ESO-1, WT1, AFP, CA125, TAG-72, B7-H3, PD-L1, Trop2, Trop3, or CD95. Similarly, the invention encompasses TAAs typically associated with hematological malignancies, such as CD19, CD20, CD22, CD30, CD33, CD123, BCMA, FLT3, CLL-1, and SLAMF7.

It is preferred that the extracellular antigen-binding domain (i) is selected from antibodies specifically binding to CD19, ErbB2, or BCMA.

In preferred embodiments, the extracellular antigen-binding domain (i) is selected from single-chain antibody fragments, especially scFvs, specifically binding to CD19, ErbB2, or BCMA, optionally having the amino acid sequence of SEQ ID NO: 10, SEQ ID NO: 12, or SEQ ID NO: 14, or a functional variant thereof.

In further preferred embodiments, the chimeric antigen receptor has an intracellular CD4 co-stimulatory domain (iii) of SEQ ID NOs: 21 or 27 or a functional variant thereof having at least 75%, at least 80%, 81%, 82%, 83%, 84%, 85%, 86%, 87%, 88%, 89%, 90%, 90%, 91%, 92%, 93%, 94%, 95%, 96%, 97%, 98%, 99%, sequence identity or homology, preferably identity.

The chimeric antigen receptor (CAR) of the invention comprises preferably the amino acid sequences of any one of SEQ ID NOs 28-39, and 42-45 or functional variants thereof having at least 75%, at least 80%, 81%, 82%, 83%, 84%, 85%, 86%, 87%, 88%, 89%, 90%, 90%, 91%, 92%, 93%, 94%, 95%, 96%, 97%, 98%, 99%, sequence identity or homology, preferably identity, and optionally variants thereof lacking the Myc tag.

The chimeric antigen receptor (CAR) of the invention preferably comprises in an N- to C-terminal arrangement,
an antigen-binding scFv fragment,
linked to a hinge region of SEQ ID NO: 17,
linked to a transmembrane domain of SEQ ID NO: 19,
linked to an intracellular signaling moiety comprising
   an ordered arrangement of activating and co-stimulatory domains,
   domain 1 of SEQ ID NOs: 21 or 27,
   domain 2 of SEQ ID NO: 23, and
   domain 3 of SEQ ID NO: 25,
   including all possible permutations of this intracellular domain order,
   including those where domain 2 is absent, and
wherein the scFv is selected from SEQ ID NOs: 10, 12, or 14, and
wherein the CAR comprises an N-terminal located leader peptide of SEQ ID NO: 6, and/or a Myc-tag of SEQ ID NO: 8,
or functional variants thereof having at least 75%, at least 80%, 81%, 82%, 83%, 84%, 85%, 86%, 87%, 88%, 89%, 90%, 90%, 91%, 92%, 93%, 94%, 95%, 96%, 97%, 98%, 99%, sequence identity or homology, preferably identity, to said CAR.

The chimeric antigen receptor (CAR) of the invention comprises preferably the amino acid sequences of any one of SEQ ID NOs 28-39, and 42-45, and/or functional variants thereof, optionally incl. variants lacking the Myc tag, comprising a sequence:
**(A)** that is at least 75% at least 80%, 81%, 82%, 83%, 84%, 85%, 86%, 87%, 88%, 89%, 90%, 90%, 91%, 92%, 93%, 94%, 95%, 96%, 97%, 98%, 99% identical to any one of SEQ ID NOs 28-39, and 42-45, or
**(B)** that is at least 75% at least 80%, 81%, 82%, 83%, 84%, 85%, 86%, 87%, 88%, 89%, 90%, 90%, 91%, 92%, 93%, 94%, 95%, 96%, 97%, 98%, 99% identical to the transmembrane and cytoplasmic domains of any one of SEQ ID NOs 28-39, and 42-45.

In a specific embodiment, the chimeric antigen receptor (CAR) comprising an intracellular CD4 domain is configured to provide the co-stimulatory CD4 activity on a separate polypeptide rather than as part of the CAR itself. The separate polypeptide chain is not specifically limited but is designed to include a functional CD4 co-stimulatory domain as described herein. This configuration reflects the surprising finding that the mere spatial proximity of the CD4 co-stimulatory domain to the CAR is sufficient to exert the advantageous effects described herein, such as increasing CAR-T cell persistence and/or efficiency.

The functional co-stimulatory CD4 domain on a separate polypeptide can be provided using known methods, for example, by co-expression of an encoding nucleic acid. This nucleic acid may be transiently introduced into the host cell via transfection, using the herein disclosed methods for CAR delivery, such as utilizing a viral delivery system, and/or may be integrated into the genome of the host cell.

In a specific embodiment, the co-stimulatory CD4 domain on a separate polypeptide replaces the CD4 co-stimulatory domain within the CAR, e.g. the CAR is devoid of a CD4 co-stimulatory domain.

In another preferred embodiment, the co-stimulatory CD4 activity on a separate polypeptide is provided in addition to the CD4 co-stimulatory domain within the CAR. Further preferred is a method for improving the persistence and/or efficiency of host cells comprising a CAR, comprising providing a nucleic acid encoding a functional co-stimulatory CD4 domain on a polypeptide that is not a polypeptide encoding a CAR. The method optionally comprises simultaneously providing a nucleic acid encoding a CAR, preferably a CAR according to the first aspect of the invention.

Further disclosed is a kit or a composition for improving the persistence and/or efficiency of host cells comprising a CAR, comprising (i) a nucleic acid encoding a functional co-stimulatory CD4 domain as part of a peptide chain that is not a polypeptide comprising a CAR, together with (ii) suitable buffers and/or excipients. The kit or composition optionally comprises a nucleic acid encoding a CAR, preferably a CAR according to the first aspect of the invention.

In a further aspect, the invention relates to nucleic acids encoding the herein disclosed CAR fusion proteins, wherein the nucleic acids are not limited and include nucleic acids in linear or circular form, for example, single-stranded RNA such as mRNA, as well as single- or double-stranded DNA.

Disclosed are, among others, nucleic acids encoding the CAR fusion proteins according to SEQ ID NOs: 28-39, and 42-45, or variants of these nucleic acids with at least 75%, at least 80%, 81%, 82%, 83%, 84%, 85%, 86%, 87%, 88%, 89%, 90%, 91%, 92%, 93%, 94%, 95%, 96%, 97%, 98%, or 99% sequence identity or homology, preferably identity.

Preferred are nucleic acids according to SEQ ID NOs: 1-4 or variants with at least 75%, at least 80%, 81%, 82%, 83%, 84%, 85%, 86%, 87%, 88%, 89%, 90%, 91%, 92%, 93%, 94%, 95%, 96%, 97%, 98%, or 99% sequence identity or homology, preferably identity.

Preferred is a nucleic acid encoding a CAR fusion protein as disclosed herein, wherein the nucleic acid comprises:
**(A)** a nucleotide sequence encoding the intracellular domain of CD4 according to SEQ ID NO: 21 or SEQ ID NO: 27, or a sequence that has at least 75% least 80%, 81%, 82%, 83%, 84%, 85%, 86%, 87%, 88%, 89%, 90%, 91%, 92%, 93%, 94%, 95%, 96%, 97%, 98%, or 99% nucleotide sequence identity thereto, or
**(B)** a nucleotide sequence encoding the transmembrane and cytoplasmic domains of any one of SEQ ID NOs 28-39, and 42-45, or a sequence that has at least 75% least 80%, 81%, 82%, 83%, 84%, 85%, 86%, 87%, 88%, 89%, 90%, 91%, 92%, 93%, 94%, 95%, 96%, 97%, 98%, or 99% nucleotide sequence identity thereto, or
**(C)** a nucleotide sequence encoding any one of SEQ ID NOs: 28-39, and 42-45 or functional variants thereof, in particular nucleic acids of SEQ ID NOs: 1-4, or nucleic acids having at least 75% least 80%, 81%, 82%, 83%, 84%, 85%, 86%, 87%, 88%, 89%, 90%, 91%, 92%, 93%, 94%, 95%, 96%, 97%, 98%, or 99% nucleotide sequence identity thereto.

To exert a therapeutic effect, the CARs disclosed usually have to be integrated into the cell membrane of a cytotoxic cell in a spatially correct manner. This is preferably achieved through the expression of the encoding nucleic acids in the respective cytotoxic cells.

The expression of nucleic acids encoding the herein disclosed CAR can be achieved by operably linking the DNA encoding the CAR to a promoter (either constitutive or inducible), optionally through integration of the nucleic acid into an expression cassette e.g. of a vector or a plasmid. The promoter can be any promoter of interest, including viral promoters. Optionally, an enhancer might be included in the construct. The cassettes can be suitable for replication and integration in either prokaryotes or eukaryotes.

In a further aspect, the invention relates to a recombinant expression vector comprising a nucleic acid encoding the herein disclosed CAR.

For the expression of the CAR in a cytotoxic host cell, preferably a T cell or NK cell, the encoding nucleic acid must be either permanently integrated into the genome or transiently introduced into the cell. Any suitable coding nucleic acid may be introduced into the cell, for example, via electroporation. Particularly suitable forms include plasmid-based DNA, RNA, or mRNA constructs. Preferred embodiments encompass vectors and plasmids with integrated expression cassettes, as well as viral vectors.

When using viral vectors, the selection can take into account the specific requirements of the system. Lentiviral vectors are capable of stable integration into the host genome and support long-term expression, making them suitable for both dividing and non-dividing cells. Retroviral vectors also enable stable expression but are primarily effective in dividing cells. Adenoviral vectors facilitate transient but highly efficient gene expression and are characterized by their large packaging capacity, whereas Sendai viruses offer transient expression without genomic integration and high transduction efficiency in primary cells. Adeno-associated viruses (AAV) are notable for their low immunogenicity and their ability to support both transient and stable expression, though they are limited by their smaller packaging capacity.

For stable expression, lentiviral or retroviral vectors are preferred. For transient expression, adenoviral vectors or Sendai viruses are particularly suitable. For specific requirements, such as low immunogenicity, adeno-associated viruses (AAV) are advantageous.

In the context of the invention, lentiviral vector systems are in particular preferred.

In a further aspect the invention relates to an isolated mammalian cell comprising a CAR as disclosed herein, a vector for the expression of said CAR, or a mammalian cell that has been engineered or genetically modified to express the CAR-encoding nucleic acids disclosed herein. These cells are designed to express the CAR in a targeted and efficient manner, ensuring their suitability for therapeutic applications.

The mammalian cell provided according to the invention is preferably selected from the group consisting of αβ T cells, γδ T cells, natural killer (NK) cells, natural killer T (NKT) cells, cytotoxic T lymphocytes (CTLs), regulatory T cells, macrophages, or combinations thereof. Of course, also any populations of such cells are comprised.

Among these, T cells are the best-characterized cell type in the development of therapeutic CARs, owing to their well-established role in adaptive immunity and their ability to mediate highly specific cytotoxic responses against target cells.

NK cells, in contrast, are particularly advantageous due to their excellent safety profile. Unlike T cells, NK cells are less likely to induce severe side effects such as cytokine release syndrome, making them especially suitable for allogeneic therapies. NKT cells combine features of T cells and NK cells, providing hybrid functionality that includes both cytotoxicity and immune modulation.

Regulatory T cells are valuable for suppressing autoimmune reactions and maintaining immune tolerance, while macrophages hold promise in targeting solid tumors through phagocytosis and modulation of the tumor microenvironment.

The diversity of suitable cytotoxic host cell types underscores the broad therapeutic applicability of the herein disclosed CARs.

In a further aspect the invention relates to pharmaceutical composition comprising the herein disclosed CAR, the herein disclosed nucleic acids or recombinant vectors, or the here disclosed isolated mammalian cell or population thereof, and a pharmaceutically acceptable carrier and/or excipient.

Examples of suitable carriers and excipients for formulating compositions comprising the herein disclosed CAR, the herein disclosed nucleic acids or recombinant vectors, or the here disclosed isolated mammalian cell or population thereof include saline and aqueous buffer solutions and are well known in the art.

Typically, the pharmaceutical composition is adapted for parenteral administration, e.g., for intravenous infusion or injection, which is highly preferred for compositions containing cells. In further embodiments, the pharmaceutical composition is adapted for local administration, such as intratumoral injection, or for regional perfusion to target specific tissues or organs.

The determination of dosage and frequency of administration lies within the professional judgment of the treating physician and may vary depending on the patient's condition, disease stage, and individual response. Typically, the pharmaceutical composition is administered as a single infusion of a therapeutically effective dose, but in some cases, additional doses may be required to achieve or maintain the desired therapeutic effect. For example, the composition may be administered in one or more treatment cycles, with intervals of several weeks to months between infusions, depending on the therapeutic response and tolerability. Re-administration may be necessary in cases of disease progression or suboptimal initial response.

### MEDICAL APPLICATIONS

According to a further aspect of the invention the herein disclosed subject matter, in particular the CAR, the nucleic acid encoding the CAR, the cells comprising the CAR as well as disclosed pharmaceutical compositions is used in medicine, including human and veterinary medicine, particularly in human medicine.

Due to the modular design of the CAR, the therapeutic spectrum of said CAR depends solely on the selected external antigen-binding domain. The skilled artisan will select this domain as needed from the vast array of already characterized antigen-binding domains known in the art. Accordingly, subject matter disclosed herein is particularly suitable for therapies aimed at the elimination or reduction of a specific, typically pathological cell population. For example, they are suitable for the treatment of proliferative diseases, both solid and hematological in nature, provided these are characterized by distinctive surface antigens (TAA).

Relevant diseases are referred to in the context of the invention as diseases "associated with the expression" of a specific antigen. This term encompasses overexpression, decreased/inhibited expression, or even unchanged expression of a target antigen compared to a non-pathological condition, depending on the target and/or the disease to be treated.

"Suitable for therapy" within the meaning of the present invention refers to the use of the entities for the treatment, amelioration, and prevention of the respective disease.

The subject matter of the invention, in particular, provides promising treatment options for individuals who have relapsed or become refractory after prior treatment with one or more medications - often considered the standard of care - including those targeting the same antigen.

### Erb2 (HER2/neu) related disorders

Within the meaning of the present invention, the subject matter described herein with an Erb2-binding antibody domain is suitable for therapeutic application in proliferative or malignant disorders associated with Erb2 expression.

In particular solid tumors such as prostate cancer, lung cancer, non-small cell lung cancer (NSCLC), melanoma, lymphoma, breast cancer, head and neck cancer, renal cell carcinoma (RCC), ovarian cancer, kidney cancer, urinary bladder cancer, uterine cancer, cervical cancer, ovarian cancer, liver cancer, stomach cancer, colon cancer, rectal cancer, oral cavity cancer, pharynx cancer, pancreatic cancer, thyroid cancer, skin cancer, brain cancer are characterized to comprise HER2 expressing cells.

### BCMA related disorders

Within the meaning of the present invention, the subject matter described herein with an BCMA-binding antibody domain is suitable for therapeutic application in proliferative or malignant disorders associated with BCMA expression.

Hematological tumors such as Multiple Myeloma (MM), Waldenström's Macroglobulinemia, Chronic Lymphocytic Leukemia (CLL), Plasma Cell Leukemia, and Burkitt's Lymphoma are characterized by the presence of BCMA-expressing cells (B-cells).

Additionally, autoreactive B-cells in diseases such as Systemic Lupus Erythematosus (SLE) and Sjögren's Syndrome are also characterized by BCMA expression, presenting a potential therapeutic option for BCMA-directed CAR-T cell therapy.

### CD19 related disorders

Within the meaning of the present invention, the subject matter described herein with an CD19-binding antibody domain is suitable for therapeutic application in proliferative or malignant disorders associated with CD19 expression.

In particular, hematological tumors such as Acute Lymphoblastic Leukemia (ALL), Chronic Lymphocytic Leukemia (CLL), Diffuse Large B-Cell Lymphoma (DLBCL), Follicular Lymphoma (FL), Mantle Cell Lymphoma (MCL), Burkitt Lymphoma, Hairy Cell Leukemia (HCL), Marginal Zone Lymphoma (MZL), and Waldenström's Macroglobulinemia (WM) are characterized by the presence of CD19-expressing cells (B-cells).

The ubiquitous expression of CD19 on B-cells provides a strong rationale for developing targeted therapies in autoimmune diseases characterized by autoreactive B-cells. Thus CD19-targeted therapies hold significant promise for addressing autoimmune conditions such as Systemic Lupus Erythematosus (Guffroy et.al 2024), Rheumatoid Arthritis (RA), Myasthenia gravis (Haghikia et al 2023) Sjögren's Syndrome, and Multiple Sclerosis (Fischbach et al. 2024) offering a highly specific and effective treatment option.

The skilled person understands that the aforementioned HER2 (Erb2), BCMA and CD19 related disorders merely illustrate a preliminary list of options for respective targeting treatments and is certainly aware of further diseases that benefit from therapies specifically targeting HER2, BCMA, and CD19.

The following examples illustrate the invention with further details and serve to provide a better understanding of the invention but are not intended to limit the invention and its applicability in any way, especially not to the embodiments presented therein.

An aspect of the invention relates to a method of treating, preventing, or ameliorating a condition or disorder associated with the expression of an antigen, preferably a tumor antigen, in a subject, the method comprising administering to the subject an effective amount of the CAR as herein described, and/or the nucleic acid or recombinant vector as herein described, and/or the isolated mammalian cell or population thereof as herein described, and/or the pharmaceutical composition as herein described.

### Examples:

### Example 1: Design and preparation of DNA constructs for the demonstration of the invention

For the preparation of DNA constructs coding CARs that comprise CD4 domain, standard molecular biology techniques were employed, including the chemical transformation of competent E. *coli* cells, isolation of plasmid DNA, polymerase chain reaction (PCR), Gibson assembly, ligation, determination of nucleic acid concentration, agarose gel electrophoresis of DNA, isolation of DNA fragments from agarose gels, chemical synthesis of DNA, digestion of DNA with restriction enzymes, digestion of plasmid vectors, ligation of DNA fragments, and large-scale purification of plasmid DNA. These procedures are well known to those skilled in the art and are described in detail in molecular biology handbooks.

All plasmids, completed constructs and partial constructs were transformed into bacterium *E. coli* NEB stable by chemical transformation. Plasmids for transfection into the cell line Lenti-X 293T were isolated using GeneJet Plasmid DNA Isolation Kit (InVitrogen).

### Example 2: Construction of CAR T Cells with CD4 Costimulatory Domain

Viral Production: Lentiviral particles were produced by transiently transfecting plasmids into Lenti-X 293T cells. Cells were seeded at a density of 5 × 10⁶ cells per plate. After 24 hours, the cells were transfected with a mixture of 10 µg of the pCDH vector expressing the protein of interest, 5 µg of the pMDLg/pRRe packaging vector (Addgene plasmid #12251), 2.5 µg of the VSV-G envelope vector (Addgene plasmid #12259), and 5 µg of the pRSV-Rev vector (Addgene plasmid #12253) using polyethylenimine MAX (PEI MAX). Forty-eight hours post-transfection, the supernatant containing lentiviral particles was harvested and filtered through a 0.45-µm filter. The filtered supernatant was subsequently ultracentrifuged at 4°C for 2 hours at 100,000g using ultracentrifuge tubes. The resulting viral pellet was resuspended in phosphate-buffered saline (PBS) and stored at -80°C until use.

CAR T Cell Construction: Primary human T cells were isolated from healthy donors, adhering to ethical and safety standards. Peripheral blood mononuclear cells (PBMCs) were isolated from healthy donor buffy coats via Lymphoprep density gradient centrifugation. CD3+ T cells were negatively selected using the Pan T Cell Isolation Kit (Miltenyi Biotec) according to the manufacturer's instructions. Isolated T cells were cultured in RPMI medium supplemented with 10% heat-inactivated fetal bovine serum (FBS), 25 µL/mL ImmunoCult Human CD3/CD28 T cell activator, and 50 U/mL human recombinant IL-2 for 48 hours before transduction.

T cells were transduced with lentiviral particles at a multiplicity of infection (MOI) of 10-15. Following transduction, T cells were cultured and expanded for at least 5-10 days in the presence of 50 U/mL human recombinant IL-2. The efficiency of CAR expression was assessed via flow cytometry by measuring Myc-tag expression on the cell surface. Human T cells from multiple healthy donors were utilized for this process, and all samples were handled in compliance with ethical and biosafety regulations.

### Example 3: In Vitro Cytotoxicity Assays

The cytotoxicity of modified CAR T cells was evaluated against target antigens CD19, Her2, and BCMA to demonstrate their effectiveness relative to the second-generation CAR T wild type.

CAR T cells and non-modified T cells were washed to remove human IL-2 and subsequently resuspended in RPMI medium. Target cancer cell lines expressing the antigens of interest were co-cultured with CAR T cells at the indicated effector-to-target (E:T) ratios. RAJI-FLuc cells were utilized as targets for CD19 and BCMA, while MCF7-ErbB2-Fluc cells were used to assess the targeting of Her2. The co-cultures were incubated under standard conditions and after 24 hours. Prior to bioluminescence measurement, D-luciferin was added to a final concentration of 150 µg/mL. Bioluminescence imaging (BLI) was performed using the IVIS Lumina Series III system to measure signal intensity. Data acquisition and analysis were conducted using Living Image 4.5.2 software. The percentage of specific target cell lysis was calculated from average radiance values (ARV) using the formula: % specific lysis = 100 × (spontaneous death ARV - test ARV)/(spontaneous death ARV - background ARV).

Results: The killing efficacy of CAR T cells modified to include a CD4 costimulatory domain was assessed in comparison to standard CAR T cells with 41BB and CD3ζ domains (Figure 2). Targeting CD19 on RAJI cells demonstrated enhanced killing efficacy for CD4-modified CAR T cells, with CD4 costimulation significantly improving lysis compared to CAR T cells containing the 41BB domain alone (Figure 2A). Targeting Her2 on MCF7-ErbB2+ cells showed increased cytotoxicity of CD4-modified CAR T cells relative to control CAR constructs (Figure 2B). Targeting BCMA on RAJI cells also exhibited superior lysis rates with CD4 costimulation, further supporting the utility of this modification (Figure 2C).

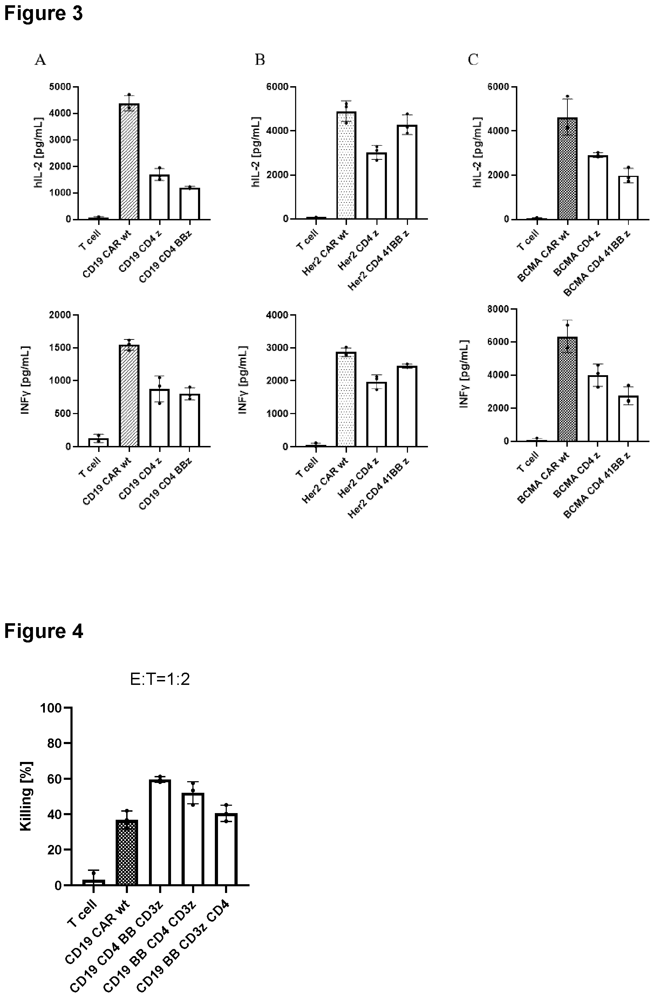

In additional studies, several alternative placements of the CD4 domain within the CAR construct were tested. The results indicated that all placements outperformed CAR wt but the construct with CD4 41BBz placement was the best-performing, demonstrating superior cytotoxicity across the conditions tested (Figure 4).

These results demonstrate that the inclusion of the CD4 costimulatory domain, either alone or in combination with 41BB, enhances CAR T cell-mediated cytotoxicity across multiple tumor antigen targets. The best-performing construct was CD4-41BB, which is consistent across all antigens.

### Example 4: Cytokine Production Analysis

The release of cytokines, including IL-2 and IFN-γ, was measured to evaluate immune activation mediated by CAR T cells incorporating a CD4 costimulatory domain in comparison to CAR wt constructs.

Supernatants from co-cultures of CAR T cells and target cells, previously used for cytotoxicity assays, were collected after centrifugation at 1800 rpm for 5 minutes to pellet the cells. The concentrations of human IL-2 and IFN-γ in the supernatants were quantified using commercially available ELISA kits (Thermo Fisher Scientific), in accordance to the manufacturer's protocols. Washing steps during ELISA procedures were performed using a HydroSpeed plate washer (Tecan). Absorbance was measured using a SynergyMx microplate reader (BioTek), and endpoint readings were analyzed to determine cytokine concentrations.

Results: The levels of cytokine secretion in response to different target antigens are shown in Figure 3. When targeting CD19 on RAJI cells, CAR wt constructs exhibited higher levels of both IL-2 and IFN-γ secretion compared to CD4-modified CAR constructs, including CD4z and CD4-41BBz (Figure 3A). In targeting Her2 on MCF7-ErbB2+ cells, CAR wt constructs also demonstrated significantly greater cytokine production relative to the CD4-modified CAR T cells (Figure 3B). Similarly, in targeting BCMA on RAJI cells, CAR wt constructs induced markedly higher IL-2 and IFN-γ secretion compared to CD4z and CD4 41BBz constructs (Figure 3C).

These findings indicate that CAR T cells incorporating the CD4 costimulatory domain exhibit reduced cytokine secretion compared to CAR wt constructs, highlighting a distinct functional difference in immune activation between these designs.

### Example 5: Animal Model Studies (in Vivo Tumor Killing)

The therapeutic efficacy of CAR T cells incorporating a CD4 costimulatory domain was evaluated in vivo using a xenograft mouse model of human lymphoma.

Pathogen-free SCID mice were bred and housed under the controlled environmental conditions, including a 12-hour light/dark cycle, relative humidity between 40-60%, and a temperature range of 20-24°C. Animals were maintained in individually ventilated cages (IVC) with access to standard laboratory chow and water ad libitum. Health certificates accompanied all animals, with their microbiological status confirmed using the FELASA-recommended Mouse Vivum panel (QM Diagnostics).

For the in vivo study, 8- to 12-week-old SCID mice were selected. Tumor xenografts were established by intravenously injecting 1 × 10⁶ RAJI-FLuc cells per mouse. On day 3 post-tumor cell injection, CAR T cells were administered intravenously at a dose of 5 × 10⁶ cells per mouse. Tumor burden was monitored weekly through bioluminescent imaging. Before imaging, the mice were injected intraperitoneally with D-luciferin (150 mg/kg; Sigma) and anesthetized using isoflurane inhalation. Ten minutes after luciferin administration, in vivo bioluminescent imaging was performed using the IVIS Lumina Series III system (PerkinElmer). Data acquisition and analysis were conducted using Living Image 4.5.2 software (PerkinElmer) with appropriate background subtraction.

Following a defined experimental period or upon reaching ethical endpoints, the mice were humanely sacrificed, and relevant data were collected.

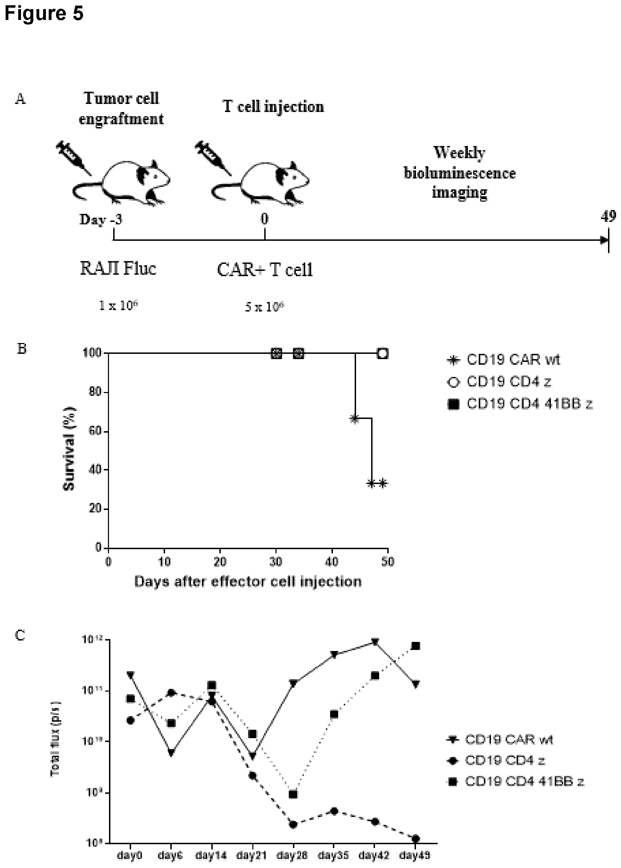

Results: The experimental scheme shown in Figure 5A depicts the timeline of tumor establishment and CAR T cell administration. The Kaplan-Meier survival curve demonstrates the prolonged survival of mice treated with CD4-modified CAR T cells compared to the control group. In the case of CD19 CD4z and CD19 CD4 41BBz, all five mice in each group survived. However, in the control group with clinically approved CD19 CAR wt, only three mice survived (Figure 5B)._Total flux (photons/second) was measured as a marker of tumor burden. CD4-modified CAR T cells exhibited enhanced tumor clearance over time, as evidenced by a reduced bioluminescence signal compared to CAR wt. The best-performing construct was CD19 CD4z (Figure 5C).

These findings confirm that CAR T cells incorporating a CD4 costimulatory domain effectively control tumor growth and improve survival in vivo.

### Example 6: Sequences of the invention

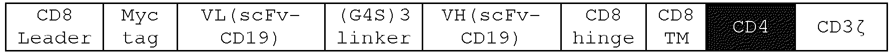

**>SEQ_ID_1** 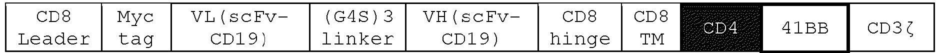 
gccctgcctgtgacagctctgctgctgcctctggccctgctgctgcatgccgccagacccgagcagaagctgatc
agcgaggaggacctggacattcaaatgactcaaacaacttcatctttgtctgcaagcctgggagaccgcgtgaca
ataagctgccgagccagtcaagatatcagcaaatatctcaattggtatcagcaaaagcctgatgggactgttaaa
ttgttgatataccatacgagtcgcctccacagcggagttccttctaggttttctggatcaggctccgggaccgac
tactccctcacgattagcaatcttgagcaggaggatatagccacatatttttgtcaacaaggaaacacacttccc
tacaccttcggtgggggaacgaaattggaaattactggtggcggcgggtctggaggtggcggtagtggaggcggg
ggctctgaagtcaagcttcaagaatcaggtccgggacttgtagcgccaagtcagtctctttccgtaacttgtacc
gttagtggggtttccttgccagattacggagtaagttggattcgccaaccgcccagaaagggcttggaatggttg
ggtgttatctggggaagcgaaacaacatactataacagcgcactcaagtctcgcctcacgattattaaagataac
tctaaatctcaggtgttcctcaaaatgaactcattgcaaacggacgacactgccatctattactgtgcaaaacac
tattactacggcggctcttacgctatggattactggggccagggaacttcagtgacagtttctagtacaaccacc
cctgcccctagacctccaaccccagcccctacaatcgccagccagcctctgtctctgaggcccgaggcttgtaga
ccagctgctggcggagccgtgcacaccagaggactggatttcgcctgcgacatctacatctgggcccctctggcc
ggcacatgtggcgtgctgctgctgagcctcgtgatcaccctgtactgccggtgcagacaccgcaggagacaagca
gaaagactcagccaaataaagagactgctctctgagaagaagacatgccagtgtcctcacagattccagaaaaca
tgcagccccataagagtgaagttcagcagatccgccgacgcccctgcctaccagcagggacagaaccagctgtac
aacgagctgaacctgggcagacgggaagagtacgacgtgctggacaagcggagaggccgggatcctgaaatgggc
ggcaagcccagacggaagaacccccaggaaggcctgtataacgaactgcagaaagacaagatggccgaggcctac
agcgagatcggaatgaagggcgagcggagaagaggcaagggccacgatggcctgtaccagggcctgagcaccgcc
accaaggacacctatgacgccctgcacatgcaggctttgccgcctaga
**>SEQ_ID_2** 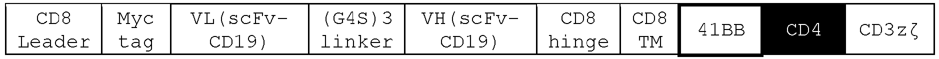 
gccctgcctgtgacagctctgctgcctctggccctgctgctgcatgccgccagacccgagcagaagctgatc
agcgaggaggacctggacattcaaatgactcaaacaacttcatctttgtctgcaagcctgggagaccgcgtgaca
ataagctgccgagccagtcaagatatcagcaaatatctcaattggtatcagcaaaagcctgatgggactgttaaa
ttgttgatataccatacgagtcgcctccacagcggagttccttctaggttttctggatcaggctccgggaccgac
tactccctcacgattagcaatcttgagcaggaggatatagccacatatttttgtcaacaaggaaacacacttccc
tacaccttcggtgggggaacgaaattggaaattactggtggcggcgggtctggaggtggcggtagtggaggcggg
ggctctgaagtcaagcttcaagaatcaggtccgggacttgtagcgccaagtcagtctctttccgtaacttgtacc
gttagtggggtttccttgccagattacggagtaagttggattcgccaaccgcccagaaagggcttggaatggttg
ggtgttatctggggaagcgaaacaacatactataacagcgcactcaagtctcgcctcacgattattaaagataac
tctaaatctcaggtgttcctcaaaatgaactcattgcaaacggacgacactgccatctattactgtgcaaaacac
tattactacggcggctcttacgctatggattactggggccagggaacttcagtgacagtttctagtacaaccacc
cctgcccctagacctccaaccccagcccctacaatcgccagccagcctctgtctctgaggcccgaggcttgtaga
ccagctgctggcggagccgtgcacaccagaggactggatttcgcctgcgacatctacatctgggcccctctggcc
ggcacatgtggcgtgctgctgctgagcctcgtgatcaccctgtactgccggtgcagacaccgcaggagacaagca
gaaagactcagccaaataaagagactgctctctgagaagaagacatgccagtgtcctcacagattccagaaaaca
tgcagccccataaagcggggcagaaagaaactgctgtacatctttaagcagcccttcatgcggcccgtgcagacc
acccaggaagaggacggctgctcctgcagattccccgaggaagaagaaggcggctgcgagctgagagtgaagttc
agcagatccgccgacgcccctgcctaccagcagggacagaaccagctgtacaacgagctgaacctgggcagacgg
gaagagtacgacgtgctggacaagcggagaggccgggatcctgaaatgggcggcaagcccagacggaagaacccc
caggaaggcctgtataacgaactgcagaaagacaagatggccgaggcctacagcgagatcggaatgaagggcgag
cggagaagaggcaagggccacgatggcctgtaccagggcctgagcaccgccaccaaggacacctatgacgccctg
cacatgcaggctttgccgcctaga
**>SEQ_ID_3** 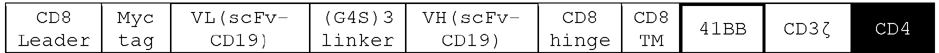 
gccctgcctgtgacagctctgctgctgcctctggccctgctgctgcatgccgccagacccgagcagaagctgatc
agcgaggaggacctggacattcaaatgactcaaacaacttcatctttgtctgcaagcctgggagaccgcgtgaca
ataagctgccgagccagtcaagatatcagcaaatatctcaattggtatcagcaaaagcctgatgggactgttaaa
ttgttgatataccatacgagtcgcctccacagcggagttccttctaggttttctggatcaggctccgggaccgac
tactccctcacgattagcaatcttgagcaggaggatatagccacatatttttgtcaacaaggaaacacacttccc
tacaccttcggtgggggaacgaaattggaaattactggtggcggcgggtctggaggtggcggtagtggaggcggg
ggctctgaagtcaagcttcaagaatcaggtccgggacttgtagcgccaagtcagtctctttccgtaacttgtacc
gttagtggggtttccttgccagattacggagtaagttggattcgccaaccgcccagaaagggcttggaatggttg
ggtgttatctggggaagcgaaacaacatactataacagcgcactcaagtctcgcctcacgattattaaagataac
tctaaatctcaggtgttcctcaaaatgaactcattgcaaacggacgacactgccatctattactgtgcaaaacac
tattactacggcggctcttacgctatggattactggggccagggaacttcagtgacagtttctagtacaaccacc
cctgcccctagacctccaaccccagcccctacaatcgccagccagcctctgtctctgaggcccgaggcttgtaga
ccagctgctggcggagccgtgcacaccagaggactggatttcgcctgcgacatctacatctgggcccctctggcc
ggcacatgtggcgtgctgctgctgagcctcgtgatcaccctgtactgcaagcggggcagaaagaaactgctgtac
atctttaagcagcccttcatgcggcccgtgcagaccacccaggaagaggacggctgctcctgcagattccccgag
gaagaagaaggcggctgcgagctgcggtgcagacaccgcaggagacaagcagaaagactcagccaaataaagaga
ctgctctctgagaagaagacatgccagtgtcctcacagattccagaaaacatgcagccccataagagtgaagttc
agcagatccgccgacgcccctgcctaccagcagggacagaaccagctgtacaacgagctgaacctgggcagacgg
gaagagtacgacgtgctggacaagcggagaggccgggatcctgaaatgggcggcaagcccagacggaagaacccc
caggaaggcctgtataacgaactgcagaaagacaagatggccgaggcctacagcgagatcggaatgaagggcgag
cggagaagaggcaagggccacgatggcctgtaccagggcctgagcaccgccaccaaggacacctatgacgccctg
cacatgcaggctttgccgcctaga
**>SEQ_ID_4** 
gccctgcctgtgacagctctgctgctgcctctggccctgctgctgcatgccgccagacccgagcagaagctgatc
agcgaggaggacctggacattcaaatgactcaaacaacttcatctttgtctgcaagcctgggagaccgcgtgaca
ataagctgccgagccagtcaagatatcagcaaatatctcaattggtatcagcaaaagcctgatggactgttaaa
ttgttgatataccatacgagtcgcctccacagcggagttccttctaggttttctggatcaggctccgggaccgac
tactccctcacgattagcaatcttgagcaggaggatatagccacatattttgtcaacaaggaaacacacttccc
tacaccttcggtggggaacgaaattggaaattactggtggcggcgggtctggaggtggcggtagtggaggcggg
ggctctgaagtcaagcttcaagaatcaggtccgggacttgtagcgccaagtcagtctctttccgtaacttgtacc
gttagtggggtttccttgccagattacggagtaagttggattcgccaaccgcccagaaagggcttggaatggttg
ggtgttatctggggaagcgaaacaacatactataacagcgcactcaagtctcgcctcacgattattaaagataac
tctaaatctcaggtgttcctcaaaatgaactcattgcaaacggacgacactgccatctattactgtgcaaaacac
tattactacggcggctcttacgctatggattactggggccagggaacttcagtgacagtttctagtacaaccacc
cctgcccctagacctccaaccccagcccctacaatcgccagccagcctctgtctctgaggcccgaggcttgtaga
ccagctgctggcggagccgtgcacaccagaggactggatttcgcctgcgacatctacatctggcccctctggcc
ggcacatgtggcgtgctgctgctgagcctcgtgatcaccctgtactgcaagcggggcagaaagaaactgctgtac
atctttaagcagcccttcatgcggcccgtgcagaccacccaggaagaggacggctgctcctgcagattccccgag
gaagaagaaggcggctgcgagctgagagtgaagttcagcagatccgccgacgcccctgcctaccagcagggacag
aaccagctgtacaacgagctgaacctgggcagacgggaagagtacgacgtgctggacaagcggagaggccgggat
cctgaaatgggcggcaagcccagacggaagaacccccaggaaggcctgtataacgaactgcagaaagacaagatg
gccgaggcctacagcgagatcggaatgaagggcgagcggagaagaggcaagggccacgatggcctgtaccagggc
ctgagcaccgccaccaaggacacctatgacgccctgcacatgcaggctttgccgcctagacggtgcagacaccgc
 
aggagacaagcagaaagactcagccaaataaagagactgctctctgagaagaagacatgccagtgtcctcacaga
ttccagaaaacatgcagccccata
 **CD8 LEADER**
**>SEQ_ID_5**
   gccctgcctgtgacagctctgctgctgcctctggccctgctgctgcatgccgccagaccc
**>SEQ_ID_6**
   ALPVTALLLPLALLLHAARP
   **MYC TAG**
**>SEQ_ID_7**
   gagcagaagctgatcagcgaggaggacctg
**>SEQ_ID_8**
   EQKLISEEDL
**Anti CD19 scFv (VL-(G4S)3 linker-VH)**
   **>SEQ_ID_9** 
gacattcaaatgactcaaacaacttcatctttgtctgcaagcctgggagaccgcgtgacaataagctgccgagcc
agtcaagatatcagcaaatatctcaattggtatcagcaaaagcctgatgggactgttaaattgttgatataccat
acgagtcgcctccacagcggagttccttctaggttttctggatcaggctccgggaccgactactccctcacgatt
agcaatcttgagcaggaggatatagccacatatttttgtcaacaaggaaacacacttccctacaccttcggtggg
ggaacgaaattggaaattactggtggcggcgggtctggaggtggcggtagtggaggcgggggctctgaagtcaag
cttcaagaatcaggtccgggacttgtagcgccaagtcagtctctttccgtaacttgtaccgttagtggggtttcc
ttgccagattacggagtaagttggattcgccaaccgcccagaaagggcttggaatggttgggtgttatctgggga
agcgaaacaacatactataacagcgcactcaagtctcgcctcacgattattaaagataactctaaatctcaggtg
ttcctcaaaatgaactcattgcaaacggacgacactgccatctattactgtgcaaaacactattactacggcggc
tcttacgctatggattactggggccagggaacttcagtgacagtttctagt
**>SEQ_ID 10** 
DIQMTQTTSSLSASLGDRVTISCRASQDISKYLNWYQQKPDGTVKLLIYHTSRLHSGVPSRFSGSGSGTDYSLTI
SNLEQEDIATYFCQQGNTLPYTFGGGTKLEITGGGGSGGGGSGGGGSEVKLQESGPGLVAPSQSLSVTCTVSGVS
LPDYGVSWIRQPPRKGLEWLGVIWGSETTYYNSALKSRLTIIKDNSKSQVFLKMNSLQTDDTAIYYCAKHYYYGG
SYAMDYWGQGTSVTVSS
**Anti Her2 (Erb2) scFv (VL-linker-VH)**
   **>SEQ_ID_11** 
gacatccagatgacccagagtcccagcagtctgagcgctagtgtaggcgaccgcgttaccatcacatgcagagca
tctcaagatgtgaatacagcagttgcgtggtatcaacagaaacctggtaaagcccctaaactgctcatctatagt
gcgtcctttctgtatagtggcgtcccctcacgcttcagcggctcacgctctgggacagactttaccttgaccatc
tcttcccttcagcctgaggactttgccacctattattgtcagcaacattacaccacaccaccaacattcgggcag
gggacgaaagtagagataaaaaggacgggctctacaagtgggtctggaaaaccgggctcaggggaaggatctgag
gtgcaactcgttgaaagtggcggcggattggtccagccggggggatccttgcgccttagctgtgctgcatcagga
tttaatatcaaggacacttacatccactgggtaaggcaagcccctggaaaggggcttgagtgggtagcccggatc
taccctacaaacggttatacccgctacgcggacagcgtgaaaggccgcttcacaatttcagccgatacttctaaa
aacactgcgtacctccaaatgaactcactgcgggcggaagatactgcagtttattactgtagcagatggggtggg
gatgggttttatgccatggatgtatgggggcagggaacacttgtaaccgtttctagc
**>SEQ_ID_12** 
DIQMTQSPSSLSASVGDRVTITCRASQDVNTAVAWYQQKPGKAPKLLIYSASFLYSGVPSRFSGSRSGTDFTLTI
SSLQPEDFATYYCQQHYTTPPTFGQGTKVEIKRTGSTSGSGKPGSGEGSEVQLVESGGGLVQPGGSLRLSCAASG
FNIKDTYIHWVRQAPGKGLEWVARIYPTNGYTRYADSVKGRFTISADTSKNTAYLQMNSLRAEDTAVYYCSRWGG
DGFYAMDVWGQGTLVTVSS
**Anti BCMA scFv (VH PMC306-(G4S)3 linker-VL PMC306)**
   **>SEQ_ID_13** caggtgcagctggtgcagagcggcgcggaagtgaaaaaaccgggcagcagcgtgaaagtgagctgcaaagcgagc 
ggctatacctttaccagctatgtgatgcattgggtgcgccaggcgccgggccagggcctggaatggatgggctat
attattccgtataacgatgcgaccaaatataacgaaaaatttaaaggccgcgtgaccattaccgcggataaaagc
accagcaccgcgtatatggaactgagcagcctgcgcagcgaagataccgcggtgtattattgcgcgcgctataac
tatgatggctattttgatgtgtggggccagggcaccctggtgaccgtgagcagcggtggcggcgggtctggaggt
ggcggtagtggaggcgggggctctgaaattgtgctgacccagagcccggcgaccctgagcctgagcccgggcgaa
cgcgcgaccctgagctgccgcgcgagccagagcattagcgattatctgcattggtatcagcagaaaccgggccag
gcgccgcgcctgctgatttattatgcgagccagagcattaccggcattccggcgcgctttagcggcagcggcagc
ggcaccgattttaccctgaccattagcagcctggaaccggaagattttgcggtgtattattgccagaacggccat
agctttccgccgacctttggcggcggcaccaaagtggaaattaaa
 
>SEQ_ID_14
QVQLVQSGAEVKKPGSSVKVSCKASGYTFTSYVMHWVRQAPGQGLEWMGYIIPYNDATKYNEKFKGRVTITADKS
TSTAYMELSSLRSEDTAVYYCARYNYDGYFDVWGQGTLVTVSSGGGGSGGGGSGGGGSEIVLTQSPATLSLSPGE
RATLSCRASQSISDYLHWYQQKPGQAPRLLIYYASQSITGIPARFSGSGSGTDFTLTISSLEPEDFAVYYCQNGH
SFPPTFGGGTKVEIK
 **(G₄S)₃-Linker**
**>SEQ**_**ID**_**15**
   GGGSGGGSGGGS
**CD8 HINGE**
**>SEQ**_**ID**_**16** 
acaaccacccctgcccctagacctccaaccccagcccctacaatcgccagccagcctctgtctctgaggcccgag
gcttgtagaccagctgctggcggagccgtgcacaccagaggactggatttcgcctgcgacatctac
**>SEQ**_**ID**_**17**
   TTTPAPRPPTPAPTIASQPLSLRPEACRPAAGGAVHTRGLDFACDIY
**CD8 TRANSMEMBRANE DOMAIN**
**>SEQ**_**ID**_**18**
   atctgggcccctctggccggcacatgtggcgtgctgctgctgagcctcgtgatcaccctgtactgc
**>SEQ**_**ID**_**19**
   IWAPLAGTCGVLLLSLVITLYC
**CD4 DOMAIN (costimulation domain R421** - **I458)**
   **>SEQ**_**ID**_**20** 
cggtgcagacaccgcaggagacaagcagaaagactcagccaaataaagagactgctctctgagaagaagacatgc
cagtgtcctcacagattccagaaaacatgcagccccata
**>SEQ_ID_21**
   RCRHRRRQAERLSQIKRLLSEKKTCQCPHRFQKTCSPI
**41BB (co-stimulatory domain)**
   **>SEQ**_**ID**_**22** 
aagcggggcagaaagaaactgctgtacatctttaagcagcccttcatgcggcccgtgcagaccacccaggaagag
gacggctgctcctgcagattccccgaggaagaagaaggcggctgcgagctg
**>SEQ_ID_23**
   KRGRKKLLYIFKQPFMRPVQTTQEEDGCSCRFPEEEEGGCEL
**CD3 ζ**
   **>SEQ**_**ID**_**24** 
agagtgaagttcagcagatccgccgacgcccctgcctaccagcagggacagaaccagctgtacaacgagctgaac
ctgggcagacgggaagagtacgacgtgctggacaagcggagaggccgggatcctgaaatgggcggcaagcccaga
cggaagaacccccaggaaggcctgtataacgaactgcagaaagacaagatggccgaggcctacagcgagatcgga
atgaagggcgagcggagaagaggcaagggccacgatggcctgtaccagggcctgagcaccgccaccaaggacacc
tatgacgccctgcacatgcaggctttgccgcctaga
 
>SEQ_ID_25
RVKFSRSADAPAYQQGQNQLYNELNLGRREEYDVLDKRRGRDPEMGGKPRRKNPQEGLYNELQKDKMAEAYSEIG
MKGERRRGKGHDGLYQGLSTATKDTYDALHMQALPPR
**CD4 (Uniprot ID: P01730)**
   >**SEQ_ID_26**|P01730|CD4_HUMAN T-cell surface glycoprotein CD4 length=458|intracellular domain= AA 419-458 
MNRGVPFRHLLLVLQLALLPAATQGKKVVLGKKGDTVELTCTASQKKSIQFHWKNSNQIKILGNQGSFLTKGPSK
LNDRADSRRSLWDQGNFPLIIKNLKIEDSDTYICEVEDQKEEVQLLVFGLTANSDTHLLQGQSLTLTLESPPGSS
PSVQCRSPRGKNIQGGKTLSVSQLELQDSGTWTCTVLQNQKKVEFKIDIVVLAFQKASSIVYKKEGEQVEFSFPL
AFTVEKLTGSGELWWQAERASSSKSWITFDLKNKEVSVKRVTQDPKLQMGKKLPLHLTLPQALPQYAGSGNLTLA
LEAKTGKLHQEVNLVVMRATQLQKNLTCEVWGPTSPKLMLSLKLENKEAKVSKREKAVWVLNPEAGMWQCLLSDS
GQVLLESNIKVLPTWSTPVQPMALIVLGGVAGLLLFIGLGIFFCVRCRHRRRQAERMSQIKRLLSEKKTCQCPHR
FQKTCSPI
**CD4 DOMAIN (costimulation domain R421** - **I458 according to Uniprot P01730) >SEQ_ID_27**
   RCRHRRRQAERMSQIKRLLSEKKTCQCPHRFQKTCSPI
**CAR-CD19_CD4_z**
   **>SEQ_ID_28** 
ALPVTALLLPLALLLHAARPEQKLISEEDLDIQMTQTTSSLSASLGDRVTISCRASQDISKYLNWYQQKPDGTVK
LLIYHTSRLHSGVPSRFSGSGSGTDYSLTISNLEQEDIATYFCQQGNTLPYTFGGGTKLEITGGGGSGGGGSGGG
GSEVKLQESGPGLVAPSQSLSVTCTVSGVSLPDYGVSWIRQPPRKGLEWLGVIWGSETTYYNSALKSRLTIIKDN
SKSQVFLKMNSLQTDDTAIYYCAKHYYYGGSYAMDYWGQGTSVTVSSTTTPAPRPPTPAPTIASQPLSLRPEACR
PAAGGAVHTRGLDFACDIYIWAPLAGTCGVLLLSLVITLYCRCRHRRRQAERLSQIKRLLSEKKTCQCPHRFQKT
CSPIRVKFSRSADAPAYQQGQNQLYNELNLGRREEYDVLDKRRGRDPEMGGKPRRKNPQEGLYNELQKDKMAEAY
SEIGMKGERRRGKGHDGLYQGLSTATKDTYDALHMQALPPR
**CAR- CD19_CD4_41BB_z**
   **>SEQ_ID_29** 
ALPVTALLLPLALLLHAARPEQKLISEEDLDIQMTQTTSSLSASLGDRVTISCRASQDISKYLNWYQQKPDGTVK
LLIYHTSRLHSGVPSRFSGSGSGTDYSLTISNLEQEDIATYFCQQGNTLPYTFGGGTKLEITGGGGSGGGGSGGG
GSEVKLQESGPGLVAPSQSLSVTCTVSGVSLPDYGVSWIRQPPRKGLEWLGVIWGSETTYYNSALKSRLTIIKDN
SKSQVFLKMNSLQTDDTAIYYCAKHYYYGGSYAMDYWGQGTSVTVSSTTTPAPRPPTPAPTIASQPLSLRPEACR
PAAGGAVHTRGLDFACDIYIWAPLAGTCGVLLLSLVITLYCRCHRRRQAERLSQIKRLLSEKKTCQCPHRFQKT
CSPIKRGRKKLLYIFKQPFMRPVQTTQEEDGCSCRFPEEEEGGCELRVKFSRSADAPAYQQGQNQLYNELNLGRR
EEYDVLDKRRGRDPEMGGKPRRKNPQEGLYNELQKDKMAEAYSEIGMKGERRRGKGHDGLYQGLSTATKDTYDAL
HMQALPPR
**CAR-CD19_41BB_CD4_z**
   **>SEQ_ID_30** 
ALPVTALLLPLALLLHAARPEQKLISEEDLDIQMTQTTSSLSASLGDRVTISCRASQDISKYLNWYQQKPDGTVK
LLIYHTSRLHSGVPSRFSGSGSGTDYSLTISNLEQEDIATYFCQQGNTLPYTFGGGTKLEITGGGGSGGGGSGGG
GSEVKLQESGPGLVAPSQSLSVTCTVSGVSLPDYGVSWIRQPPRKGLEWLGVIWGSETTYYNSALKSRLTIIKDN
SKSQVFLKMNSLQTDDTAIYYCAKHYYYGGSYAMDYWGQGTSVTVSSTTTPAPRPPTPAPTIASQPLSLRPEACR
PAAGGAVHTRGLDFACDIYIWAPLAGTCGVLLLSLVITLYCKRGRKKLLYIFKQPFMRPVQTTQEEDGCSCRFPE
EEEGGCELRCRHRRRQAERLSQIKRLLSEKKTCQCPHRFQKTCSPIRVKFSRSADAPAYQQGQNQLYNELNLGRR
EEYDVLDKRRGRDPEMGGKPRRKNPQEGLYNELQKDKMAEAYSEIGMKGERRRGKGHDGLYQGLSTATKDTYDAL
HMQALPPR
**CAR-CD19_CD4_z_41BB >SEQ_ID_31** 
ALPVTALLLPLALLLHAARPEQKLISEEDLDIQMTQTTSSLSASLGDRVTISCRASQDISKYLNWYQQKPDGTVK
LLIYHTSRLHSGVPSRFSGSGSGTDYSLTISNLEQEDIATYFCQQGNTLPYTFGGGTKLEITGGGGSGGGGSGGG
GSEVKLQESGPGLVAPSQSLSVTCTVSGVSLPDYGVSWIRQPPRKGLEWLGVIWGSETTYYNSALKSRLTIIKDN
SKSQVFLKMNSLQTDDTAIYYCAKHYYYGGSYAMDYWGQGTSVTVSSTTTPAPRPPTPAPTIASQPLSLRPEACR
PAAGGAVHTRGLDFACDIYIWAPLAGTCGVLLLSLVITLYCRCRHRRRQAERLSQIKRLLSEKKTCQCPHRFQKT
CSPIRVKFSRSADAPAYQQGQNQLYNELNLGRREEYDVLDKRRGRDPEMGGKPRRKNPQEGLYNELQKDKMAEAY
SEIGMKGERRRGKGHDGLYQGLSTATKDTYDALHMQALPPRKRGRKKLLYIFKQPFMRPVQTTQEEDGCSCRFPE
EEEGGCEL
**CAR-HER2**_**CD4**_**z**
   >SEQ_ID_32 
ALPVTALLLPLALLLHAARPEQKLISEEDLDIQMTQSPSSLSASVGDRVTITCRASQDVNTAVAWYQQKPGKAPK
LLIYSASFLYSGVPSRFSGSRSGTDFTLTISSLQPEDFATYYCQQHYTTPPTFGQGTKVEIKRTGSTSGSGKPGS
GEGSEVQLVESGGGLVQPGGSLRLSCAASGFNIKDTYIHWVRQAPGKGLEWVARIYPTNGYTRYADSVKGRFTIS
ADTSKNTAYLQMNSLRAEDTAVYYCSRWGGDGFYAMDVWGQGTLVTVSSTTTPAPRPPTPAPTIASQPLSLRPEA
CRPAAGGAVHTRGLDFACDIYIWAPLAGTCGVLLLSLVITLYCRCRHRRRQAERLSQIKRLLSEKKTCQCPHRFQ
KTCSPIRVKFSRSADAPAYQQGQNQLYNELNLGRREEYDVLDKRRGRDPEMGGKPRRKNPQEGLYNELQKDKMAE
AYSEIGMKGERRRGKGHDGLYQGLSTATKDTYDALHMQALPPR
**CAR-HER2_CD4_41BB_z**
   **>SEQ_ID_33** 
ALPVTALLLPLALLLHAARPEQKLISEEDLDIQMTQSPSSLSASVGDRVTITCRASQDVNTAVAWYQQKPGKAPK
LLIYSASFLYSGVPSRFSGSRSGTDFTLTISSLQPEDFATYYCQQHYTTPPTFGQGTKVEIKRTGSTSGSGKPGS
GEGSEVQLVESGGGLVQPGGSLRLSCAASGFNIKDTYIHWVRQAPGKGLEWVARIYPTNGYTRYADSVKGRFTIS
ADTSKNTAYLQMNSLRAEDTAVYYCSRWGGDGFYAMDVWGQGTLVTVSSTTTPAPRPPTPAPTIASQPLSLRPEA
CRPAAGGAVHTRGLDFACDIYIWAPLAGTCGVLLLSLVITLYCRCHRRRQAERLSQIKRLLSEKKTCQCPHRFQ
KTCSPIKRGRKKLLYIFKQPFMRPVQTTQEEDGCSCRFPEEEEGGCELRVKFSRSADAPAYQQGQNQLYNELNLG
RREEYDVLDKRRGRDPEMGGKPRRKNPQEGLYNELQKDKMAEAYSEIGMKGERRRGKGHDGLYQGLSTATKDTYD
ALHMQALPPR
**CAR-HER2_41BB_CD4_z**
   **>SEQ_ID_34** 
ALPVTALLLPLALLLHAARPEQKLISEEDLDIQMTQSPSSLSASVGDRVTITCRASQDVNTAVAWYQQKPGKAPK
LLIYSASFLYSGVPSRFSGSRSGTDFTLTISSLQPEDFATYYCQQHYTTPPTFGQGTKVEIKRTGSTSGSGKPGS
GEGSEVQLVESGGGLVQPGGSLRLSCAASGFNIKDTYIHWVRQAPGKGLEWVARIYPTNGYTRYADSVKGRFTIS
ADTSKNTAYLQMNSLRAEDTAVYYCSRWGGDGFYAMDVWGQGTLVTVSSTTTPAPRPPTPAPTIASQPLSLRPEA
CRPAAGGAVHTRGLDFACDIYIWAPLAGTCGVLLLSLVITLYCKRGRKKLLYIFKQPFMRPVQTTQEEDGCSCRF
PEEEEGGCELRCRHRRRQAERLSQIKRLLSEKKTCQCPHRFQKTCSPIRVKFSRSADAPAYQQGQNQLYNELNLG
RREEYDVLDKRRGRDPEMGGKPRRKNPQEGLYNELQKDKMAEAYSEIGMKGERRRGKGHDGLYQGLSTATKDTYD
ALHMQALPPR
**CAR-HER2_CD4_z_41BB**
   **>SEQ_ID_35** 
ALPVTALLLPLALLLHAARPEQKLISEEDLDIQMTQSPSSLSASVGDRVTITCRASQDVNTAVAWYQQKPGKAPK
LLIYSASFLYSGVPSRFSGSRSGTDFTLTISSLQPEDFATYYCQQHYTTPPTFGQGTKVEIKRTGSTSGSGKPGS
GEGSEVQLVESGGGLVQPGGSLRLSCAASGFNIKDTYIHWVRQAPGKGLEWVARIYPTNGYTRYADSVKGRFTIS
ADTSKNTAYLQMNSLRAEDTAVYYCSRWGGDGFYAMDVWGQGTLVTVSSTTTPAPRPPTPAPTIASQPLSLRPEA
CRPAAGGAVHTRGLDFACDIYIWAPLAGTCGVLLLSLVITLYCRCRHRRRQAERLSQIKRLLSEKKTCQCPHRFQ
KTCSPIRVKFSRSADAPAYQQGQNQLYNELNLGRREEYDVLDKRRGRDPEMGGKPRRKNPQEGLYNELQKDKMAE
AYSEIGMKGERRRGKGHDGLYQGLSTATKDTYDALHMQALPPRKRGRKKLLYIFKQPFMRPVQTTQEEDGCSCRF
PEEEEGGCEL
**CAR-BCMA_CD 4_z**
   **>SEQ_ID_36** 
ALPVTALLLPLALLLHAARPEQKLISEEDLQVQLVQSGAEVKKPGSSVKVSCKASGYTFTSYVMHWVRQAPGQGL
EWMGYIIPYNDATKYNEKFKGRVTITADKSTSTAYMELSSLRSEDTAVYYCARYNDGYFDVWGQGTLVTVSSGG
GGSGGGGSGGGGSEIVLTQSPATLSLSPGERATLSCRASQSISDYLHWYQQKPGQAPRLLIYYASQSITGIPARF
SGSGSGTDFTLTISSLEPEDFAVYYCQNGHSFPPTFGGGTKVEIKTTTPAPRPPTPAPTIASQPLSLRPEACRPA
AGGAVHTRGLDFACDIYIWAPLAGTCGVLLLSLVITLYCRCRHRRRQAERLSQIKRLLSEKKTCQCPHRFQKTCS
PIRVKFSRSADAPAYQQGQNQLYNELNLGRREEYDVLDKRRGRDPEMGGKPRRKNPQEGLYNELQKDKMAEAYSE
IGMKGERRRGKGHDGLYQGLSTATKDTYDALHMQALPPR
**CAR-BCMA**_**CD4**_**41BB**_**z**
   **>SEQ_ID_37** 
ALPVTALLLPLALLLHAARP EQKLISEEDL
QVQLVQSGAEVKKPGSSVKVSCKASGYTFTSYVMHWVRQAPGQGLEWMGYIIPYNDATKYNEKFKGRVTITADKS
TSTAYMELSSLRSEDTAVYYCARYNYDGYFDVWGQGTLVTVSSGGGGSGGGGSGGGGSEIVLTQSPATLSLSPGE
RATLSCRASQSISDYLHWYQQKPGQAPRLLIYYASQSITGIPARFSGSGSGTDFTLTISSLEPEDFAVYYCQNGH
SFPPTFGGGTKVEIK
TTTPAPRPPTPAPTIASQPLSLRPEACRPAAGGAVHTRGLDFACDIYIWAPLAGTCGVLLLSLVITLYC
RCRHRRRQAERLSQIKRLLSEKKTCQCPHRFQKTCSPI
KRGRKKLLYIFKQPFMRPVQTTQEEDGCSCRFPEEEEGGCEL
RVKFSRSADAPAYQQGQNQLYNELNLGRREEYDVLDKRRGRDPEMGGKPRRKNPQEGLYNELQKDKMAEAYSEIG
MKGERRRGKGHDGLYQGLSTATKDTYDALHMQALPPR
**CAR-BCMA_41BB_CD4**_**z**
   >**SEQ_ID_38** 
ALPVTALLLPLALLLHAARP EQKLISEEDL
QVQLVQSGAEVKKPGSSVKVSCKASGYTFTSYVMHWVRQAPGQGLEWMGYIIPYNDATKYNEKFKGRVTITADKS
TSTAYMELSSLRSEDTAVYYCARYNYDGYFDVWGQGTLVTVSSGGGGSGGGGSGGGGSEIVLTQSPATLSLSPGE
RATLSCRASQSISDYLHWYQQKPGQAPRLLIYYASQSITGIPARFSGSGSGTDFTLTISSLEPEDFAVYYCQNGH
SFPPTFGGGTKVEIKTTTPAPRPPTPAPTIASQPLSLRPEACRPAAGGAVHTRGLDFACDIYIWAPLAGTCGVLL
LSLVITLYC
KRGRKKLLYIFKQPFMRPVQTTQEEDGCSCRFPEEEEGGCELRCRHRRRQAERLSQIKRLLSEKKTCQCPHRFQK
TCSPIRVKFSRSADAPAYQQGQNQLYNELNLGRREEYDVLDKRRGRDPEMGGKPRRKNPQEGLYNELQKDKMAEA
YSEIGMKGERRRGKGHDGLYQGLSTATKDTYDALHMQALPPR
**CAR-BCMA_CD4_z_41BB**
   **>SEQ_ID_39** 
ALPVTALLLPLALLLHAARP EQKLISEEDL
QVQLVQSGAEVKKPGSSVKVSCKASGYTFTSYVMHWVRQAPGQGLEWMGYIIPYNDATKYNEKFKGRVTITADKS
TSTAYMELSSLRSEDTAVYYCARYNYDGYFDVWGQGTLVTVSSGGGGSGGGGSGGGGSEIVLTQSPALSLSPGER
ATLSCRASQSISDYLHWYQQKPGQAPRLLIYYASQSITGIPARFSGSGSGTDFTLTISSLEPEDFAVYYCQNGHS
FPPTFGGGTKVEIKTTTPAPRPPTPAPTIASQPLSLRPEACRPAAGGAVHTRGLDFACDIYIWAPLAGTCGVLLL
SLVITLYCRCRHRRRQAERLSQIKRLLSEKKTCQCPHRFQKTCSPIRVKFSRSADAPAYQQGQNQLYNELNLGRR
EEYDVLDKRRGRDPEMGGKPRRKNPQEGLYNELQKDKMAEAYSEIGMKGERRRGKGHDGLYQGLSTATKDTYDAL
HMQALPPRKRGRKKLLYIFKQPFMRPVQTTQEEDGCSCRFPEEEEGGCEL
**Anti GD2 scFv (VL-(G4S)3 linker-VH)**
   **>SEQ_ID_40** 
gacgtcgtcatgacgcaaactccgctctccctcccagtatctttgggagaccaggcgagtatttcatgtcgaagc
agccaaagtttggtgcatcgcaacggaaatacgtacctgcactggtatcttcaaaagccgggccagagccctaaa
cttctgattcacaaggttagtaaccgattttctggtgtgcctgaccgcttctctgggtcaggctccggtacggac
 
tttacgctcaaaataagccgggtcgaggccgaagacctgggcgtctatttttgctctcagtcaactcatgttcct
ccgttgacatttggggcaggcactaaattggaactcaagagagcgggcggaggcggatctgggggaggaggcagt
ggaggcggagggtctgaagtgcaattgctgcaaagtgggccagagttggaaaagcctggagcttccgtaatgatt
tcctgtaaggcctcaggaagctcctttaccggatataatatgaattgggtacgacagaacatcggtaaaagtctt
gaatggatcggtgcaatcgacccgtactacggaggtactagttataaccaaaaattcaaaggtcgcgccactctt
actgtcgataagtcctctagcacagcctatatgcacctcaagagcctgacgtcagaagactcagccgtatactat
tgcgtgagcggtatggagtattggggccaagggacatctgtaactgtctctagc
**>SEQ ID 41** 
DVVMTQTPLSLPVSLGDQASISCRSSQSLVHRNGNTYLHWYLQKPGQSPKLLIHKVSNRFSGVPDRFSGSGSGTD
FTLKISRVEAEDLGVYFCSQSTHVPPLTFGAGTKLELKRAGGGGSGGGGSGGGGSEVQLLQSGPELEKPGASVMI
SCKASGSSFTGYNMNWVRQNIGKSLEWIGAIDPYYGGTSYNQKFKGRATLTVDKSSSTAYMHLKSLTSEDSAVYY
CVSGMEYWGQGTSVTVSS
**CAR-GD2_CD4_z**
   **>SEQ_ID_42** 
ALPVTALLLPLALLLHAARP EQKLISEEDL
DVVMTQTPLSLPVSLGDQASISCRSSQSLVHRNGNTYLHWYLQKPGQSPKLLIHKVSNRFSGVPDRFSGSGSGTD
FTLKISRVEAEDLGVYFCSQSTHVPPLTFGAGTKLELKRAGGGGSGGGGSGGGGSEVQLLQSGPELEKPGASVMI
SCKASGSSFTGYNMNWVRQNIGKSLEWIGAIDPYYGGTSYNQKFKGRATLTVDKSSSTAYMHLKSLTSEDSAVYY
CVSGMEYWGQGTSVTVSSTTTPAPRPPTPAPTIASQPLSLRPEACRPAAGGAVHTRGLDFACDIYIWAPLAGTCG
VLLLSLVITLYCRCRHRRRQAERLSQIKRLLSEKKTCQCPHRFQKTCSPIRVKFSRSADAPAYQQGQNQLYNELN
LGRREEYDVLDKRRGRDPEMGGKPRRKNPQEGLYNELQKDKMAEAYSEIGMKGERRRGKGHDGLYQGLSTATKDT
YDALHMQALPPR
**CAR-GD2_CD4_41BB_z**
   **>SEQ_ID_43** 
ALPVTALLLPLALLLHAARP EQKLISEEDL
DVVMTQTPLSLPVSLGDQASISCRSSQSLVHRNGNTYLHWYLQKPGQSPKLLIHKVSNRFSGVPDRFSGSGSGTD
FTLKISRVEAEDLGVYFCSQSTHVPPLTFGAGTKLELKRAGGGGSGGGGSGGGGSEVQLLQSGPELEKPGASVMI
SCKASGSSFTGYNMNWVRQNIGKSLEWIGAIDPYYGGTSYNQKFKGRATLTVDKSSSTAYMHLKSLTSEDSAVYY
CVSGMEYWGQGTSVTVSSTTTPAPRPPTPAPTIASQPLSLRPEACRPAAGGAVHTRGLDFACDIYIWAPLAGTCG
VLLLSLVITLYCRCRHRRRQAERLSQIKRLLSEKKTCQCPHRFQKTCSPIKRGRKKLLYIFKQPFMRPVQTTQEE
DGCSCRFPEEEEGGCELRVKFSRSADAPAYQQGQNQLYNELNLGRREEYDVLDKRRGRDPEMGGKPRRKNPQEGL
YNELQKDKMAEAYSEIGMKGERRRGKGHDGLYQGLSTATKDTYDALHMQALPPR
**CAR-GD2_41BB_CD4_z**
   **>SEQ_ID_44** 
ALPVTALLLPLALLLHAARP EQKLISEEDL
DVVMTQTPLSLPVSLGDQASISCRSSQSLVHRNGNTYLHWYLQKPGQSPKLLIHKVSNRFSGVPDRFSGSGSGTD
FTLKISRVEAEDLGVYFCSQSTHVPPLTFGAGTKLELKRAGGGGSGGGGSGGGGSEVQLLQSGPELEKPGASVMI
SCKASGSSFTGYNMNWVRQNIGKSLEWIGAIDPYYGGTSYNQKFKGRATLTVDKSSSTAYMHLKSLTSEDSAVYY
CVSGMEYWGQGTSVTVSSTTTPAPRPPTPAPTIASQPLSLRPEACRPAAGGAVHTRGLDFACDIYIWAPLAGTCG
VLLLSLVITLYCKRGRKKLLYIFKQPFMRPVQTTQEEDGCSCRFPEEEEGGCELRCRHRRRQAERLSQIKRLLSE
KKTCQCPHRFQKTCSPIRVKFSRSADAPAYQQGQNQLYNELNLGRREEYDVLDKRRGRDPEMGGKPRRKNPQEGL
YNELQKDKMAEAYSEIGMKGERRRGKGHDGLYQGLSTATKDTYDALHMQALPPR
**CAR-GD2_CD4_z_41BB**
   **>SEQ_ID_45**

ALPVTALLLPLALLLHAARP EQKLISEEDL
DVVMTQTPLSLPVSLGDQASISCRSSQSLVHRNGNTYLHWYLQKPGQSPKLLIHKVSNRFSGVPDRFSGSGSGTD
FTLKISRVEAEDLGVYFCSQSTHVPPLTFGAGTKLELKRAGGGGSGGGGSGGGGSEVQLLQSGPELEKPGASVMI
SCKASGSSFTGYNMNWVRQNIGKSLEWIGAIDPYYGGTSYNQKFKGRATLTVDKSSSTAYMHLKSLTSEDSAVYY
CVSGMEYWGQGTSVTVSSTTTPAPRPPTPAPTIASQPLSLRPEACRPAAGGAVHTRGLDFACDIYIWAPLAGTCG
VLLLSLVITLYCRCRHRRRQAERLSQIKRLLSEKKTCQCPHRFQKTCSPIRVKFSRSADAPAYQQGQNQLYNELN
LGRREEYDVLDKRRGRDPEMGGKPRRKNPQEGLYNELQKDKMAEAYSEIGMKGERRRGKGHDGLYQGLSTATKDT
YDALHMQALPPRKRGRKKLLYIFKQPFMRPVQTTQEEDGCSCRFPEEEEGGCEL

### References

Cappell K. M., Kochenderfer J. N. A comparison of chimeric antigen receptors containing CD28 versus 41BB costimulatory domains. Nature Review Clinical Oncology, 18, 11: 715-727 (2021)
Felix Fischbach et al.; CD19-targeted chimeric antigen receptor T cell therapy in two patients with multiple sclerosis, Med, Volume 5, Issue 6, Pages 550-558.e2 (2024)
Glatzová D, Cebecauer M. Dual Role of CD4 in Peripheral T Lymphocytes. Front Immunol. Apr 2;10:618. doi: 10.3389/fimmu.2019.00618. PMID: 31001252; PMCID: PMC6454155 (2019)
Guffroy A. et al. CAR-T cells for treating systemic lupus erythematosus: A promising emerging therapy. Joint Bone Spine, Volume 91, Issue 5 (2024)
Haghikia, A. et al. Anti-CD19 CAR T cells for refractory myasthenia gravis. The Lancet Neurology, 22(12), 1104-1105 (2023)
Killeen N, Littman DR. The regulation and function of the CD4 coreceptor during T lymphocyte development. Curr Top Microbiol Immunol. 205:89-106. doi: 10.1007/978-3-642-79798-9_5. PMID: 8575199 (1996)
Maher, J., Brentjens, R., Gunset, G. et al. Human T-lymphocyte cytotoxicity and proliferation directed by a single chimeric TCRζ /CD28 receptor. Nat Biotechnol 20: 70-75 https://doi.org/10.1038/nbt0102-70 (2002)
Michel Sadelain, Renier Brentjens, Isabelle Rivière, The promise and potential pitfalls of chimeric antigen receptors, Current Opinion in Immunology, 21, 2: 215-223, https://doi.org/10.1016/j.coi.2009.02.009 (2009)
Moreno-Cortes E, Franco-Fuquen P, Garcia-Robledo JE, Forero J, Booth N, Castro JE. ICOS and OX40 tandem co-stimulation enhances CAR T-cell cytotoxicity and promotes T-cell persistence phenotype. Front Oncol. Aug 18;13:1200914. doi: 10.3389/fonc.2023.1200914. PMID: 37719008; PMCID: PMC10502212 (2023)
Peter W. Kim et al. ,A Zinc Clasp Structure Tethers Lck to T Cell Coreceptors CD4 and CD8. Science 301, 1725-1728.DOI: 10.1126/science. 1085643 (2003)
Smirnov S, Mateikovich P, Samochernykh K, Shlyakhto E. Recent advances on CAR T signaling pave the way for prolonged persistence and new modalities in clinic. Front Immunol. 22;15:1335424. doi: 10.3389/fimmu.2024.1335424. PMID: 38455066; PMCID: PMC10918004 (2024)
Van Der Stegen S. J., Hamieh M., Sadelain M. The pharmacology of secondgeneration chimeric antigen receptors. Nature Review Drug Discovery, 9, 14: 499-509 (2015)

## Claims

1. A chimeric antigen receptor (CAR) comprising an intracellular CD4 domain.

2. The chimeric antigen receptor of claim 1, wherein the intracellular CD4 domain has a length of at least 5 amino acid residues, more preferably 38 amino acid residues.

3. The chimeric antigen receptor of claim 1 or 2 comprising the components:
(i) an extracellular antigen binding domain,
(ii) a hinge and transmembrane domain,
(iii) a CD4 intracellular co-stimulatory domain, preferably a mammalian, more preferably human CD4 intracellular co-stimulatory domain, and
(iv) an activating domain,
and wherein components (i), to (iv) are preferably comprised in N- to C-terminal order.

4. The chimeric antigen receptor of any one of claims 1 to 3,
wherein the antigen binding domain (i) is a functional binding fragment of a natural binding protein, in particular a receptor or an antibody, preferably a single-chain variable fragment (scFv) of an antibody; or a short synthetic protein that binds to the selected antigen, preferably a DARPin or a designed minibinder.

5. The chimeric antigen receptor of any one of claims 1 to 4,
wherein the hinge and transmembrane domain (ii) preferably comprise the hinge and transmembrane domain of CD8-alpha, and
wherein the components (i) and (ii) are optionally joined by a short flexible amino acid linker, and/or
wherein the activating domain (iv) comprises a CD3ζ domain or a CD3ε intracellular domain or another domain that comprises two or more ITAM motifs.

6. The chimeric antigen receptor of any one of claims 1 to 5,
wherein the co-stimulatory domain (iii) comprises an additional co-stimulatory domain, fused N- or C-terminally, preferably C-terminally, to the CD4 domain, preferably a 41BB domain.

7. The chimeric antigen receptor of claim 6, wherein the activating domain (iv) is positioned between the two co-stimulatory domains of (iii), or, in configurations where the activating domain (iv) is N-terminally linked to component (ii), it is C-terminally linked to component (iii).

8. The chimeric antigen receptor of any one of claims 1 to 7,
wherein the antigen binding domain (i) specifically binds an antigen on the surface of a pathologic cell, preferably a tumor associated antigen, for example CD19, BCMA, GD2 or ErbB2.

9. The chimeric antigen receptor of any one of claims 1 to 8, wherein the intracellular CD4 co-stimulatory domain (iii) comprises the amino acid sequence of the intracellular domain of human CD4 of SEQ ID NO: 26, or the amino acid sequence of SEQ ID NO: 21 or SEQ ID NO: 27 or a functional variant thereof having at least 75% sequence identity.

10. The chimeric antigen receptor (CAR) of any one of claims 1 to 9, wherein the CAR comprises the amino acid sequences of any one of SEQ ID NOs 28-39, and 42-45 and/or functional variants thereof comprising a sequence:
**(A)** that is at least 75% identical to a sequence of any one of SEQ ID NOs 28-39, and 42-45, including variants that optionally lack the Myc tag, or
**(B)** that is at least 75% identical to the transmembrane and cytoplasmic domains of any one of SEQ ID NOs 28-39, and 42-45.

11. A nucleic acid encoding a CAR fusion protein of any one of claims 1 to 10, wherein the nucleic acid comprises:
**(A)** a nucleotide sequence encoding the intracellular domain of CD4 according to SEQ ID NO: 21 or SEQ ID NO: 27, or
**(B)** a nucleotide sequence encoding the transmembrane and cytoplasmic domains of any one of SEQ ID NOs 28-39, and 42-45, or
**(C)** a nucleotide sequence encoding any one of SEQ ID NOs: 28-39, and 42-45, in particular any one of SEQ ID NOs: 1-4, or
functional variants thereof having at least 75% sequence identity thereto.

12. A recombinant expression vector comprising the encoding nucleic acid of claim 11, wherein the expression vector is optionally a viral vector.

13. An isolated mammalian cell comprising the expression vector of claim 12 or being engineered to express the encoding nucleic acid of claim 11,
wherein the host cell is preferably selected from the group consisting of an αβ T cell, a γδ T cell, a natural killer (NK) cell, a natural killer T (NKT) cell, a cytotoxic T lymphocyte (CTL), a regulatory T cell, macrophage or combinations thereof.

14. A pharmaceutical composition comprising the CAR according to any one of claims 1-10, the nucleic acid or recombinant vector according to claims 11 to 12, or the isolated mammalian cell or population thereof according to claim 13, and a pharmaceutically acceptable carrier and/or excipient.

15. The CAR according to any one of claims 1-10, and/or the nucleic acid or recombinant vector according to claims 11 to 12, and/or the isolated mammalian cell or population thereof according to claim 13, and/or the pharmaceutical composition of claim 14, for use in treating, preventing and/or ameliorating a condition or disorder associated with the expression of an antigen, preferably a tumor antigen.
